# EUROPEAN PATENT SPECIFICATION

(11) **EP 0 814 831 B1**
(45) Date of publication and mention of the grant of the patent: **19.06.2002**
(21) Application number: 96911300.0
(22) Date of filing: 15.03.1996
(51) Int. Cl.: A61K 38/21, C07K 14/555, C12N 15/20

(54) **MEDICAMENTS FOR TREATMENT OF AUTOIMMUNE DISEASES USING INTERFERON-TAU**
MEDIKAMENTE ZUR BEHANDLUNG VON AUTOIMMUNERKRANKUNGEN MIT INTERFERON-TAU
MEDICAMENTS POUR LE TRAITEMENT DE MALADIES AUTO-IMMUNES A L'AIDE DE L'INTERFERON TAU

(30) Priority: 16.03.1995 US 406190
(43) Date of publication of application: 07.01.1998
(73) Proprietor: UNIVERSITY OF FLORIDA, Gainesville, Florida 32611-5500 (US)
(72) Inventor: SOOS, Jeanne M., Apartment 236, Gainesville, FL 32607 (US); SCHIFFENBAUER, Joel, Gainesville, FL 32607 (US); JOHNSON, Howard, Marcellus, Gainesville, FL 32606 (US)
(74) Representative: VOSSIUS & PARTNER
(86) International application number: PCT/US96/03472
(87) International publication number: WO 96/28183

(56) References cited:
- WO-A-90/09806
- WO-A-94/10313
- FASEB JOURNAL FOR EXPERIMENTAL BIOLOGY, vol. 9, no. 4, 10 March 1995, BETHESDA, MD US, page A1025 XP002010951 J.M. SOOS ET AL: "The novel Type I Interferon Tau development and superantigen reactivation of experimental allergic encephalomyelitis in mice without associated toxicity" & EXPERIMENTAL BIOLOGY 95 PART II , 9 - 13 April 1995, ATLANTA ; GEORGIA ,USA.,
- JOURNAL OF INTERFERON AND CYTOKINE RESEARCH, vol. 15, no. 1, January 1995, pages 39-45, XP002010952 J.M. SOOS ET AL: "Type I interferon inhibition of superantigen stimulation: Implications for treatment of superantigen-associated disease "
- NEUROLOGY , vol. 43, 1993, pages 655-661, XP002010953 IFNBETA MULTIPLE SCLEROSIS STUDY GROUP: "IFN beta-1b is effective in relapsing-remitting multiple sclerosis.I. Clinical results of a multicenter,randomized, double-blind, placebo controlled trial "
- JOURNAL OF IMMUNOLOGY, vol. 155, no. 5, 1 September 1995, BALTIMORE US, pages 2747-2753, XP002010954 J.M. SOOS ET AL: "The IFN pregnancy recognition hormone IFN-tau blocks both development and superantigen reactivation of experimental allergic encephalomyelitis without associated toxicity"

### SEQUENCE LISTING

(1) GENERAL INFORMATION:
   (i) APPLICANT: University of Florida
   (ii) TITLE OF INVENTION: Method for Treatment of Autoimmune Diseases
   (iii) NUMBER OF SEQUENCES: 6
   (iv) CORRESPONDENCE ADDRESS:
      (A) ADDRESSEE: Dehlinger & Associates
      (B) STREET: 350 Cambridge Ave., Suite 250
      (C) CITY: Palo Alto
      (D) STATE: CA
      (E) COUNTRY: USA
      (F) ZIP: 94306
   (v) COMPUTER READABLE FORM:
      (A) MEDIUM TYPE: Floppy disk
      (B) COMPUTER: IBM PC compatible
      {C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER:
      (B) FILING DATE: 15-MAR-1996
      (C) CLASSIFICATION:
   (vii) PRIOR APPLICATION DATA:
      (A) APPLICATION NUMBER: US 08/406,190
      (B) FILING DATE: 16-MAR-1995
   (viii) ATTORNEY/AGENT INFORMATION:
      (A) NAME: Sholtz, Charles K.
      (B) REGISTRATION NUMBER: 38,615
      (C) REFERENCE/DOCKET NUMBER: 5600-0002.41
   (ix) TELECOMMUNICATION INFORMATION:
      (A) TELEPHONE: 415-324-0880
      (B) TELEFAX: 415-324-0960
(2) INFORMATION FOR SEQ ID NO:1:
   (i) SEQUENCE CHARACTERISTICS:
      (A) LENGTH: 516 base pairs
      (B) TYPE: nucleic acid
      (C) STRANDEDNESS: double
      (D) TOPOLOGY: circular
   (ii) MOLECULE TYPE: DNA
   (iii) HYPOTHETICAL: NO
   (iv) ANTI-SENSE: NO
   (vi) ORIGINAL SOURCE:
      (A) ORGANISM: Ovis aries
      (B) STRAIN: Domestic
      (D) DEVELOPMENTAL STAGE: Blastula (blastocyst)
      (F) TISSUE TYPE: Trophectoderm
      (G) CELL TYPE: Mononuclear trophectoderm cells
   (vii) IMMEDIATE SOURCE:
      (B) CLONE: oTP-1a
   (viii) POSITION IN GENOME:
      (C) UNITS: bp
   (ix) FEATURE:
      (A) NAME/KEY: CDS
      (B) LOCATION: 1..516
   (x) PUBLICATION INFORMATION:
      (A) AUTHORS: Ott, Troy L
         Van Heeke, Gino
         Johnson, Howard M
         Bazer, Fuller W
      (B) TITLE: Cloning and Expression in Saccharomyces cerevisiae of a Synthetic Gene for the Type I Trophoblast Interferon Ovine Trophoblast Protein-1:Purification and Antiviral Activity
      (C) JOURNAL: J. Interferon Res.
      (D) VOLUME: 11
      (F) PAGES: 357-364
      (G) DATE: 1991
      (K) RELEVANT RESIDUES IN SEQ ID NO:1: FROM 1 TO 516
   (xi) SEQUENCE DESCRIPTION: SEQ ID NO:1:
   TGC TAC CTG TCG CGA AAA CTG ATG CTG GAC GCT CGA GAA AAT TTA AAA         48
   Cys Tyr Leu Ser Arg Lys Leu Met Leu Asp Ala Arg Glu Asn Leu Lys
     1               5                  10                  15
   CTG CTG GAC CGT ATG AAT CGA TTG TCT CCG CAC AGC TGC CTG CAA GAC         96
   Leu Leu Asp Arg Met Asn Arg Leu Ser Pro His Ser Cys Leu Gln Asp
               20                  25                  30
   CGG AAA GAC TTC GGT CTG CCG CAG GAA ATG GTT GAA GGT GAC CAA CTG        144
   Arg Lys Asp Phe Gly Leu Pro Gln Glu Met Val Glu Gly Asp Gln Leu
           35                  40                  45
   CAA AAA GAC CAA GCT TTC CCG GTA CTG TAT GAA ATG CTG CAG CAG TCT        192
   Gln Lys Asp Gln Ala Phe Pro Val Leu Tyr Glu Met Leu Gln Gln Ser
       50                  55                  60
   TTC AAC CTG TTC TAC ACT GAA CAT TCT TCG GCC GCT TGG GAC ACT ACT        240
   Phe Asn Leu Phe Tyr Thr Glu His Ser Ser Ala Ala Trp Asp Thr Thr
    65                  70                  75                  80
   CTT CTA GAA CAA CTG TGC ACT GGT CTG CAA CAG CAA CTG GAC CAT CTG        288
   Leu Leu Glu Gln Leu Cys Thr Gly Leu Gln Gln Gln Leu Asp His Leu
                    85                  90                  95
   GAC ACT TGC CGT GGC CAG GTT ATG GGT GAA GAA GAC TCT GAA CTG GGT        336
   Asp Thr Cys Arg Gly Gln Val Met Gly Glu Glu Asp Ser Glu Leu Gly
               100                 105                 110
   AAC ATG GAT CCG ATC GTT ACT GTT AAA AAA TAT TTC CAG GGT ATC TAC        384
   Asn Met Asp Pro Ile Val Thr Val Lys Lys Tyr Phe Gln Gly Ile Tyr
           115                 120                 125
   GAC TAC CTG CAG GAA AAA GGT TAC TCT GAC TGC GCT TGG GAA ATC GTA        432
   Asp Tyr Leu Gln Glu Lys Gly Tyr Ser Asp Cys Ala Trp Glu Ile Val
       130                 135                 140
   CGC GTT GAA ATG ATG CGG GCC CTG ACT GTG TCG ACT ACT CTG CAA AAA        480
   Arg Val Glu Met Met Arg Ala Leu Thr Val Ser Thr Thr Leu Gln Lys
   145                 150                 155                 160
   CGG TTA ACT AAA ATG GGT GGT GAC CTG AAT TCT CCG                        516
   Arg Leu Thr Lys Met Gly Gly Asp Leu Asn Ser Pro
                   165                 170
(2) INFORMATION FOR SEQ ID NO:2:
   (i) SEQUENCE CHARACTERISTICS:
      (A) LENGTH: 172 amino acids
      (B) TYPE: amino acid
      (D) TOPOLOGY: linear
   (ii) MOLECULE TYPE: protein
   (vi) ORIGINAL SOURCE:
      (C) INDIVIDUAL ISOLATE: amino acid sequence of a mature OvIFNtau protein
   (xi) SEQUENCE DESCRIPTION: SEQ ID NO:2:
   Cys Tyr Leu Ser Arg Lys Leu Met Leu Asp Ala Arg Glu Asn Leu Lys
    1               5                  10                  15
   Leu Leu Asp Arg Met Asn Arg Leu Ser Pro His Ser Cys Leu Gln Asp
               20                  25                  30
   Arg Lys Asp Phe Gly Leu Pro Gln Glu Met Val Glu Gly Asp Gln Leu
           35                  40                  45
   Gln Lys Asp Gln Ala Phe Pro Val Leu Tyr Glu Met Leu Gln Gln Ser
       50                  55                  60
   Phe Asn Leu Phe Tyr Thr Glu His Ser Ser Ala Ala Trp Asp Thr Thr
   65                  70                  75                  80
   Leu Leu Glu Gln Leu Cys Thr Gly Leu Gln Gln Gln Leu Asp His Leu
                   85                  90                  95
   Asp Thr Cys Arg Gly Gln Val Met Gly Glu Glu Asp Ser Glu Leu Gly
               100                 105                 110
   Asn Met Asp Pro Ile Val Thr Val Lys Lys Tyr Phe Gln Gly Ile Tyr
           115                 120                 125
   Asp Tyr Leu Gln Glu Lys Gly Tyr Ser Asp Cys Ala Trp Glu Ile Val
       130                 135                 140
   Arg Val Glu Met Met Arg Ala Leu Thr Val Ser Thr Thr Leu Gln Lys
   145                 150                 155                 160
   Arg Leu Thr Lys Met Gly Gly Asp Leu Asn Ser Pro
                   165                 170
(2) INFORMATION FOR SEQ ID NO:3:
   (i) SEQUENCE CHARACTERISTICS:
      (A) LENGTH: 516 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (C) INDIVIDUAL ISOLATE: synthetic nucleotide sequence encoding a mature human interferon-tau protein, HuIFNtaul.
   (xi) SEQUENCE DESCRIPTION: SEQ ID NO:3:
   TGTGACTTGT CTCAAAACCA CGTTTTGGTT GGTAGAAAGA ACTTAAGACT ACTAGACGAA        60
   ATGAGACGTC TATCTCCACG CTTCTGTCTA CAAGACAGAA AGGACTTCGC TTTGCCTCAG    120
   GAAATGGTTG AAGGTGGCCA ACTACAAGAA GCTCAAGCGA TATCTGTTTT GCACGAAATG    180
   TTGCAACAAA GCTTCAACTT GTTCCACACC GAACACTCTT CGGCCGCTTG GGACACCACC    240
   TTGTTGGAAC AGCTCAGAAC CGGTTTGCAC CAACAATTGG ACAACTTGGA TGCATGTTTG    300
   GGTCAAGTTA TGGGTGAAGA AGACTCTGCT CTCGGGAGAA CCGGTCCAAC GCTAGCTTTG    360
   AAGAGATACT TCCAAGGTAT CCACGTTTAC TTGAAGGAAA AGGGTTACTC TGACTGTGCT    420
   TGGGAAACCG TGCGTCTAGA AATCATGCGT AGCTTCTCTT CTTTGATCAG CTTGCAAGAA    480
   AGATTACGTA TGATGGACGG TGACTTGTCG AGCCCA                              516
(2) INFORMATION FOR SEQ ID NO:4:
   (i) SEQUENCE CHARACTERISTICS:
      (A) LENGTH: 172 amino acids
      (B) TYPE: amino acid
      (C) STRANDEDNESS: single
      (D) TOPOLOGY: linear
   (ii) MOLECULE TYPE: protein
   (vi) ORIGINAL SOURCE:
      (C) INDIVIDUAL ISOLATE: amino acid sequence for a mature HuIFNtau protein, HuIFNtau1.
   (xi) SEQUENCE DESCRIPTION: SEQ ID NO:4:
   Cys Asp Leu Ser Gln Asn His Val Leu Val Gly Arg Lys Asn Leu Arg
   1               5                   10                  15
   Leu Leu Asp Glu Met Arg Arg Leu Ser Pro Arg Phe Cys Leu Gln Asp
               20                  25                  30
   Arg Lys Asp Phe Ala Leu Pro Gln Glu Met Val Glu Gly Gly Gln Leu
           35                  40                  45
   Gln Glu Ala Gln Ala Ile Ser Val Leu His Glu Met Leu Gln Gln Ser
       50                  55                  60
   Phe Asn Leu Phe His Thr Glu His Ser Ser Ala Ala Trp Asp Thr Thr
   65                  70                  75                  80
   Leu Leu Glu Gln Leu Arg Thr Gly Leu His Gln Gln Leu Asp Asn Leu
                   85                  90                  95
   Asp Ala Cys Leu Gly Gln Val Met Gly Glu Glu Asp Ser Ala Leu Gly
               100                 105                 110
   Arg Thr Gly Pro Thr Leu Ala Leu Lys Arg Tyr Phe Gln Gly Ile His
           115                 120                 125
   Val Tyr Leu Lys Glu Lys Gly Tyr Ser Asp Cys Ala Trp Glu Thr Val
       130                 135                 140
   Arg Leu Glu Ile Met Arg Ser Phe Ser Ser Leu Ile Ser Leu Gln Glu
   145                 150                 155                 160
   Arg Leu Arg Met Met Asp Gly Asp Leu Ser Ser Pro
                   165                 170
(2) INFORMATION FOR SEQ ID NO:5:
   (i) SEQUENCE CHARACTERISTICS:
      (A) LENGTH: 516 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (C) INDIVIDUAL ISOLATE: HuIFNtau3, mature no leader sequence
   (ix) FEATURE:
      (A) NAME/KEY: CDS
      (B) LOCATION: 1..516
   (xi) SEQUENCE DESCRIPTION: SEQ ID NO:5:
   TGT GAC CTG TCT CAG AAC CAC GTG CTG GTT GGC AGC CAG AAC CTC AGG       48
   Cys Asp Leu Ser Gln Asn His Val Leu Val Gly Ser Gln Asn Leu Arg
     1               5                  10                  15
   CTC CTG GGC CAA ATG AGG AGA CTC TCC CTT CGC TTC TGT CTG CAG GAC       96
   Leu Leu Gly Gln Met Arg Arg Leu Ser Leu Arg Phe Cys Leu Gln Asp
                20                  25                  30
   AGA AAA GAC TTC GCT TTC CCC CAG GAG ATG GTG GAG GGT GGC CAG CTC      144
   Arg Lys Asp Phe Ala Phe Pro Gln Glu Met Val Glu Gly Gly Gln Leu
            35                  40                  45
   CAG GAG GCC CAG GCC ATC TCT GTG CTC CAC GAG ATG CTC CAG CAG AGC      192
   Gln Glu Ala Gln Ala Ile Ser Val Leu His Glu Met Leu Gln Gln Ser
        50                  55                  60
   TTC AAC CTC TTC CAC ACA GAG CAC TCC TCT GCT GCC TGG GAC ACC ACC      240
   Phe Asn Leu Phe His Thr Glu His Ser Ser Ala Ala Trp Asp Thr Thr
    65                  70                  75                  80
   CTC CTG GAG CAG CTC CGC ACT GGA CTC CAT CAG CAG CTG GAT GAC CTG      288
   Leu Leu Glu Gln Leu Arg Thr Gly Leu His Gln Gln Leu Asp Asp Leu
                    85                  90                  95
   GAT GCC TGC CTG GGG CAG GTG ACG GGA GAG GAA GAC TCT GCC CTG GGA      336
   Asp Ala Cys Leu Gly Gln Val Thr Gly Glu Glu Asp Ser Ala Leu Gly
               100                 105                 110
   AGA ACG GGC CCC ACC CTG GCC ATG AAG AGG TAT TTC CAG GGC ATC CAT      384
   Arg Thr Gly Pro Thr Leu Ala Met Lys Arg Tyr Phe Gln Gly Ile His
           115                 120                 125
   GTC TAC CTG AAA GAG AAG GGA TAT AGT GAC TGC GCC TGG GAA ATT GTC      432
   Val Tyr Leu Lys Glu Lys Gly Tyr Ser Asp Cys Ala Trp Glu Ile Val
       130                 135                 140
   AGA CTG GAA ATC ATG AGA TCC TTG TCT TCA TCA ACC AGC TTG CAC AAA      480
   Arg Leu Glu Ile Met Arg Ser Leu Ser Ser Ser Thr Ser Leu His Lys
   145                 150                 155                 160
   AGG TTA AGA ATG ATG GAT GGA GAC CTG AGC TCA CCT                      516
   Arg Leu Arg Met Met Asp Gly Asp Leu Ser Ser Pro
                   165                 170
(2) INFORMATION FOR SEQ ID NO:6:
   (i) SEQUENCE CHARACTERISTICS:
      (A) LENGTH: 172 amino acids
      (B) TYPE: amino acid
      (D) TOPOLOGY: linear
   (ii) MOLECULE TYPE: protein
   (xi) SEQUENCE DESCRIPTION: SEQ ID NO:6:
   Cys Asp Leu Ser Gln Asn His Val Leu Val Gly Ser Gln Asn Leu Arg
    1               5                  10                  15
   Leu Leu Gly Gln Met Arg Arg Leu Ser Leu Arg Phe Cys Leu Gln Asp
               20                  25                  30
   Arg Lys Asp Phe Ala Phe Pro Gln Glu Met Val Glu Gly Gly Gln Leu
           35                  40                  45
   Gln Glu Ala Gln Ala Ile Ser Val Leu His Glu Met Leu Gln Gln Ser
       50                  55                  60
   Phe Asn Leu Phe His Thr Glu His Ser Ser Ala Ala Trp Asp Thr Thr
    65                 70                  75                  80
   Leu Leu Glu Gln Leu Arg Thr Gly Leu His Gln Gln Leu Asp Asp Leu
                   85                  90                  95
   Asp Ala Cys Leu Gly Gln Val Thr Gly Glu Glu Asp Ser Ala Leu Gly
               100                 105                 110
   Arg Thr Gly Pro Thr Leu Ala Met Lys Arg Tyr Phe Gln Gly Ile His
           115                 120                 125
   Val Tyr Leu Lys Glu Lys Gly Tyr Ser Asp Cys Ala Trp Glu Ile Val
       130                 135                 140
   Arg Leu Glu Ile Met Arg Ser Leu Ser Ser Ser Thr Ser Leu His Lys
   145                 150                 155                 160
   Arg Leu Arg Met Met Asp Gly Asp Leu Ser Ser Pro
                   165                 170

## Description

### FIELD OF THE INVENTION

The present invention relates to the use of IFNτ for the manufacture of a medicament as a treatment for conditions relating to immune system hypersensitivity. More particularly, the present invention relates to the treatment of autoimmune diseases. including multiple sclerosis, rheumatoid arthritis, lupus erythematosus and type I diabetes mellitus.

### REFERENCES

Ausubel, F.M., *et al*., in CURRENT PROTOCOLS IN MOLECULAR BIOLOGY. John Wiley & Sons, Inc., Media, PA (1988).
Bartol, F.F., *et al., Biol. Reprod.* 32:681-693 (1985).
Bayne, M.L., *et al., Gene* 66:235 (1988).
Bazer, F.W., *et al., Biol. Reproduc.* (abstract only) 40(suppl):63 (1989).
Bazer, F.W., *et al*., PCT publication WO/94/10313, published 11 May, 1994.
Bazer, F.W., and Johnson, H.M., *Am. J. Reprod. Immunol.* 26:19-22 (1991).
Beames, *et al., Biotechniques* 11:378 (1991).
Bergdoll, M.S., *et al., Lancet* 1:1071-1072 (1981).
Brocke, S., *et al., Nature* 365:642-644 (1993).
Carlsson. R., and Sjogren, H.O., *Cell Immunol*. 96:175-183 (1985).
Carlsson, R., *et al., J. Immunol*. 140:2484-2488 (1988).
Charlier. M., *et al., Mol. Cell Endocrinol.* 76:161-171 (1991).
Clayman. C.B., Ed., AMERICAN MEDICAL ASSOCIATION ENCYCLOPEDIA OF MEDICINE (Random House, New York. NY). 1991.
Cross, J.C., and Roberts, R.M.. *Proc. Natl. Acad. Sci. USA* 88:3817-3821 (1991).
Day, M.J.. *et al., Clin. Immunol. Immunopathol.* 35(1):85-91 (1985).
Degre, M., *Int. J. Cancer* 14:699-703 (1974).
Ecker, D.J., *et al., J. Biol. Chem.* 264:7715-7719 (1989).
Familetti, P.C., *et al., Meth. Enzymol*. 78:387 (1981).
Feher, Z., *et al*., *Curr. Genet.* 16:461 (1989).
Fent, K., and Zbinden, G., *Trends Pharm. Sci*. 8:100-105 (1987).
Figuero, F., *et al., Immunogenetics* 15:(4):399-404 (1982).
Fleischer, B., and Schrezenmeier, H., *J. Exp. Med*. 176:1697-1707 (1988).
Fritz, R.B., *et al., J. Immunol*. 130(3):1024-1026 (1983).
Gelvin, S.B. and R.A. Schilperoot, *Plant Molecular Biology* (1988).
Gnatek, G.G., *et al., Biol. Reprod.* 41:655-664 (1989).
Godkin, J.D., *et al., J. Reprod. Fertil*. 65:141-150 (1982).
Harlow, E., *et al.,* in ANTIBODIES: A LABORATORY MANUAL, Cold Spring Harbor Laboratory Press, Cold Spring Harbor, NY (1988).
Helmer, S.D., *et al., J. Reprod. Fert*. 79:83-91 (1987).
Hitzeman, R.A., *et al*., U.S. Patent No. 4,775,622, issued October 4, 1988.
IFNβ Multiple Sclerosis Study Group, *Neurology* 43(4):655 (1993).
Imakawa, K., *et al., Nature* 330:377-379 (1987).
Imakawa, K., *et al., Mol. Endocrinol*. 3:127 (1989).
Janeway, C.A., *et al., Immunol. Rev*. 107:61-88 (1989).
Jarpe, M.A., *et al., Protein Engineering* 7:863-867 (1994).
Johnson, H.M., and Magazine, H.I., *Int. Arch. Allergy Appl. Immunol*. 87:87-90 (1988).
Johnson, H.M., *et al., FASEB J.* 5:2706-2712 (1991).
Johnson, H.M., *et al., Sci. Am.* 270(5):40-47 (1994).
Kalman, B., *et al., J. Neuroimmunol*. 45:83-88 (1993).
Kaplan, J.M., *et al., Int. J. Immunopharmacol*. 15(2):113-123 (1993).
Kemppainen, R.J., and Clark, T.P., *Vet. Clin. N. Am. Small Anim. Pract.* 24(3):467-476 (1994).
Kim, C., *et al., J. Exp. Med.* 174:1431 (1991).
Klein, J., *et al., Immunogenetics* 17:553 (1983).
Klemann, S.W., *et al., Nuc. Acids Res.* 18:6724 (1990).
Kotzin, B.L., *et al., J. Exp. Med.* 265:1237 (1987).
Kristensen, A.T., *et al., J. Vet. Intern. Med*. 8(1):36-39 (1994).
Langford, M.P., *et al., Infect. Immun*. 22:62-68 (1978).
Lider, *et al., J. Immunol*., 142:148-752 (1989).
Ludwig, D.L., *et al., Gene* 132:33 (1993).
Maniatis, T., *et al.,* in MOLECULAR CLONING: A LABORATORY MANUEL, Cold Spring Harbor Laboratory (1982).
Martal, J., *et al., J. Reprod. Fertil*. 56:63-73 (1979).
Martin, E.W., in DISPENSING OF MEDICATION: A PRACTICAL MANUAL ON THE FORMULATION AND DISPENSING OF PHARMACEUTICAL PRODUCTS (Mack Publishing Co., Easton, PA), 1976.
Mullis, K.B., U.S. Patent No. 4,683,202, issued 28 July 1987.
Mullis, K.B., *et al.,* U. S. Patent No. 4,683,195, issued 28 July 1987.
Oeda, K., *et al.,* U.S. Patent No. 4,766,068, issued August 23, 1988.
Ott, T.L., *et al., J. INF Res.* 11:357-364 (1991)
Panitch, H.S., *et al., Neurology* 37:1097-1102 (1987a).
Panitch, H.S., *et al., Lancet* i:893-895 (1987b).
Pearson, W.R. and Lipman, D.J., *PNAS* 85:2444-2448 (1988).
Pearson, W.R., *Methods in Enzymology* 183:63-98 (1990).
Pontzer, C.H., *et al., Cancer Res.* 51:5304-5307 (1991).
Powell, M.B., *et al., Int. Immunol.* 2(6):539-544 (1990).
Reilly, P.R., *et al.,* BACULOVIRUS EXPRESSION VECTORS: A LABORATORY MANUAL, 1992.
Roberts, R.M., *et al., Endocrin. Rev.* 13:432-452 (1992).
Rutter, W.J., *et al*., U.S. Patent No. 4,769,238, issued September 6, 1988.
Sambrook, J., *et al.,* in MOLECULAR CLONING: A LABORATORY MANUAL, Second Edition, Cold Spring Harbor Laboratory, Cold Spring Harbor, NY (1989).
Schiffenbauer, J., *et al., Proc. Natl. Acad. Sci. USA* 90:8543-8546 (1993).
Selmaj. K.W., and Raine, C.S., *Ann. Neurol*. 23:339-346 (1988).
Shaw, K.J., *et al., DNA* 7:117 (1988).
Shen, L.P., *et al., Sci. Sin.* 29:856 (1986).
Singer, P.A., *et al., Proc. Natl. Acad. Sci. USA* 83:7018-7022 (1986).
Smith, P.K., *et al., Anal. Biochem.* 150:76 (1985).
Soos, J.M., *et* al., *J. Neuroimmunol*. 43:39-44 (1993).
Soos, J.M., and Johnson, H.M., *J. Interferon Res.* 15:39-45 (1995).
Stewart. H.J., *et al., J. Mol. Endocrinol*. 2:65 (1989).
Vallet, J.L., *et al., Biol. Reprod.* 37:1307 (1987).
Weiner. H., *et al., Ann. Rev. Immunol.* 12:809-837 (1994).
Weinstock-Guttman, B., *et al., Ann. Neurol.* 37:7-15 (1995).
Werner. L.L., *et al., Vet. Immunol. Immunopathol.* 8(1-2):183-192 (1985).
Whaley. A.E., *et al., J. Biol. Chem.* 269(14):10864-10868 (1994).
Wraith. D.C., *et al., Cell* 59:247 (1989).
White, J., *et al., Cell* 56:27-35 (1989).
Wu, D.A., *et al., DNA* 10:201 (1991).
Zamvil. S.S., *et al., Ann. Rev. Immunol.* 8:579-621 (1990).
Zamvil. S.S.. and Steinman, *L., Ann. Rev. Immunol.* 8:579-621 (1990).

### BACKGROUND OF THE INVENTION

The immune system is the body's primary defense against diseases caused by invading organisms, such as bacteria, viruses or parasites, as well as diseases caused by abnormal growth of the body's own tissues (*i.e.,* cancerous tumors). Normally, the immune system is able to distinguish the body's normal tissues, or self, from foreign or cancerous tissue, or non-self. The loss of recognition of a particular tissue as self, and the subsequent immune response directed against that tissue, typically results in an "autoimmune response" that often has serious clinical consequences.

One specific example of such an autoimmune disease is multiple sclerosis (MS), a progressive disease of the central nervous system (CNS) in which patches of myelin (the protective covering of nerve fibers) in the brain and spinal cord are destroyed by the body's own immune system. This destruction leads to scarring and damage to the underlying nerve fibers, and may manifest itself in a variety of symptoms, depending on the parts of the brain and spinal cord that are affected. Spinal cord damage may result in tingling or numbness, as well as a heavy and/or weak feeling in the extremities. Damage in the brain may result in muscle weakness, fatigue, unsteady gain, numbness, slurred speech, impaired vision, vertigo and the like.

Current therapies for multiple sclerosis include corticosteroid drugs (to alleviate the symptoms of acute episodes), as well as other biomolecules. In particular, beta-interferon (IFNβ) has been tested and approved by the U.S. Food and Drug Administration (FDA) as an MS therapy. Unfortunately, the presently-used therapies suffer from a range of problems. The drugs are often toxic at the doses required for a maximal therapeutic effect. Further, the body may become desensitized to the drug such that higher (and more toxic) doses are required to maintain even a minimal therapeutic effect.

The present invention provides the manufacture of a medicament for a method of treatment for autoimmune diseases, such as MS, that does not have the toxic side effects associated with currently-used therapies.

### SUMMARY OF THE INVENTION

In one aspect, the present invention includes the manufacture of a medicament for a method of treating an autoimmune disease in a subject in need of such treatment. In one embodiment, the autoimmune disease is multiple sclerosis. The method includes administering, to the subject, a pharmaceutically effective amount of tau-interferon. The tau-interferon may be administered, for example, orally or via intravenous or intramuscular injection. Orally-administered IFNτ is preferably ingested by the subject. The tau interferon may be derived from (have an amino acid sequence corresponding to that of) a tau-interferon from any species that expresses tau-interferon protein (*e.g.*, ovine, bovine, goat, ox, rat, mouse or human tau-interferon).

The tau-interferon may be purified from a suitable source, produced recombinantly (*i.e*., recombinant tau-interferon), or produced synthetically. In addition, tau-interferon polypeptides (typically having between about 15 and 172 amino acids) can be used in the method of the present invention. The method of the invention may also include administering a second autoimmune disease (*e.g.*, multiple sclerosis) treatment agent before, concurrently with, or after administering tau-interferon. Exemplary second treatment agents, or medicaments, include beta-interferon and corticosteroid drugs.

In a further embodiment, the present invention includes the manufacture of a medicament for a method of treating lupus erythematosus in a subject in need of such treatment. The method includes administering, to the subject, a pharmaceutically effective amount of tau-interferon.

In another embodiment, the present invention includes the manufacture of a medicament for a method of treating type I diabetes in a subject in need of such treatment. The method includes administering, to the subject, a pharmaceutically effective amount of tau-interferon.

In a further embodiment, the present invention includes the manufacture of a medicament for a method of treating rheumatoid arthritis in a subject in need of such treatment. The method includes administering, to the subject, a pharmaceutically effective amount of tau-interferon.

It is further contemplated that tau-interferon may be useful for the manufacture of a medicament for treatment of either allograft or xenograft transplantation rejection.

In another aspect, the present invention includes an improvement in the manufacture of a medicament for a method of treating a disease condition in a mammal (*e.g.*, dog or human) responsive to treatment by interferon-tau (IFNτ). The improvement comprises orally administering a therapeutically or pharmaceutically effective amount of IFNτ. The orally-administered IFNτ is preferably ingested by the mammal. In a general embodiment, the IFNτ is orally-administered at a dosage of between about 1x10⁵ and about 1x10⁸ units per day, preferably at a dosage of between about 1x10⁶ and about 1x10⁷ units per day. The IFNτ may be, for example, ovine IFNτ (OvIFNτ), *e.g.,* a polypeptide having the sequence represented as SEQ ID NO:2, or a human IFNτ (HuIFNτ), *e.g.*, a polypeptide having the sequence represented as SEQ ID NO:4 or SEQ ID NO:6.

In one embodiment, the disease condition is an immune system disorder, such as an autoimmune disorder (*e.g*., multiple sclerosis (MS), type I (insulin dependent) diabetes mellitus, lupus erythematosus, amyotrophic lateral sclerosis, Crohn's disease, rheumatoid arthritis, stomatitis, asthma, allergies or psoriasis). MS is particularly amenable to treatment using the methods of the present invention.

In another embodiment, the disease condition is a cell proliferation disorder, such as a cancer *(e.g.,* hairy cell leukemia, Kaposi's Sarcoma, chronic myelogenous leukemia, multiple myeloma, superficial bladder cancer, skin cancer (basal cell carcinoma and malignant melanoma), renal cell carcinoma, ovarian cancer, low grade lymphocytic and cutaneous T cell lymphoma, and glioma).

In yet another embodiment, the disease condition is a viral disease *(e.g.,* hepatitis A, hepatitis B, hepatitis C, non-A, non-B, non-C hepatitis, Epstein-Barr viral infection, HIV infection, herpes virus (EB, CML, herpes simplex), papilloma, poxvirus, picorna virus, adeno virus, rhino virus, HTLV I, HTLV II, and human rotavirus).

The invention also includes the manufacture of a medicament for a method of decreasing the severity or frequency of a relapse of multiple sclerosis (MS) in a human suffering from MS, by orally administering a therapeutically or pharmaceutically effective amount of interferon-tau (IFNτ) to the human. Examples of dosages and sources of IFNτ are as presented above.

In another aspect, the invention includes the manufacture of a medicament for a method of treating a cell proliferation disorder in a subject, by orally administering a therapeutically or pharmaceutically effective amount of interferon-tau (IFNτ) to the subject. The orally-administered IFNτ is preferably ingested by the subject. Examples of cell proliferation disorders amenable to treatment, dosages, and sources of IFNτ are as presented above.

In still another aspect, the invention includes the manufacture of a medicament for a method of treating a viral disease in a subject, by orally administering a therapeutically or pharmaceutically effective amount of interferon-tau (IFNτ) to the subject. The orally-administered IFNτ is preferably ingested by the subject. Examples of viral diseases amenable to treatment, dosages, and sources of IFNτ are as presented above.

A further aspect of the invention includes the manufacture of a medicament for a method of enhancing fertility in a female mammal (*e.g.*, dog or human), by orally administering a therapeutically or pharmaceutically effective amount of interferon-tau (IFNτ) to the mammal. Examples of dosages and sources of IFNτ are as presented above.

These and other objects and features of the invention will become more fully apparent when the following detailed description is read in conjunction with the accompanying drawings.

### BRIEF DESCRIPTION OF THE FIGURES

Figure 1 shows a comparison of the toxicity of IFNβ and IFNτ.
Figure 2 shows the mean severity of experimental allergic encephalomyelitis (EAE) in New Zealand White (NZW) mice immunized with MBP in the presence and absence of IFNτ.
Figure 3 shows the effects of IFNτ on proliferation of spleen cells from MBP-immunized NZW mice.
Figures 4A, 4B, 4C, 4D, 4E and 4F are graphic depictions of superantigen reactivation of EAE in the presence and absence of IFNτ.
Figure 5 shows the effects of IFNτ on Vβ-specific T-cell activation.
Figure 6 shows the amount of OvIFNτ in NZW mouse sera after administration by either oral feeding (filled bars) or i.p. injection (open bars) as measured using an anti-viral assay.
Figures 7A, 7B and 7C show the prevention of chronic-relapsing experimental allergic encephalomyelitis (EAE) in SJL mice by orally-administered (Fig. 7C) and i.p.-injected (Fig. 7B) IFNτ as compared with mice receiving no treatment (Fig. 7A).
Figures 8A, 8B and 8C show sections of mouse spinal cord stained with cresyl violet for detection of lymphocyte infiltration from EAE-induced animals receiving either no IFNτ treatment (Fig. 8A), OvIFNτ treatment by i.p. injection (Fig. 8B) or OvIFNτ treatment by oral feeding (Fig. 8C).
Figure 9 shows induction of IL-10 by either single-dose or prolonged IFNτ treatment administered by i.p. injection or oral feeding.
Figure 10 shows relapses of EAE in SJL mice following removal of IFNτ treatment.
Figure 11 shows ELISA detection of anti-OvIFNτ antibodies in the sera of OvIFNτ-treated mice following i.p. injection or oral feeding of OvIFNτ.

### BRIEF DESCRIPON OF THE SEQUENCES

SEQ ID NO:1 is the nucleotide sequence of a synthetic gene encoding ovine interferon-τ (OvIFNτ). Also shown is the encoded amino acid sequence.

SEQ ID NO:2 is an amino acid sequence of a mature OvIFNτ protein.

SEQ ID NO:3 is a synthetic nucleotide sequence encoding a mature human interferon-τ (HuIFNτ) protein.

SEQ ID NO:4 is an amino acid sequence for a mature HuIFNτ1 protein.

SEQ ID NO:5 is the nucleotide sequence, excluding leader sequence, of genomic DNA clone HuIFNτ3, a natural HuIFNτ gene.

SEQ ID NO:6 is the predicted amino acid sequence of a mature human IFNτ protein encoded by HuIFNτ3, encoded by the sequence represented as SEQ ID NO:5.

### DETAILED DESCRIPTION OF THE INVENTION

### I. Definitions

*Interferon-*τ refers to any one of a family of interferon proteins having at least one characteristic from each of the following two groups of characteristics: (i) (a) anti-luteolytic properties, (b) anti-viral properties, (c) anti-cellular proliferation properties; and (ii) about 45 to 68% amino acid homology with α-Interferons and greater than 70% amino acid homology to known IFNτ sequences (*e.g.,* Ott, *et al.,* 1991; Helmer, *et al*., 1987; Imakawa, *et al*., 1989; Whaley, *et al*., 1994; Bazer, *et al*., 1994). Amino acid homology can be determined using, for example, the LALIGN program with default parameters. This program is found in the FASTA version 1.7 suite of sequence comparison programs (Pearson and Lipman, 1988; Pearson, 1990; program available from William R. Pearson, Department of Biological Chemistry, Box 440, Jordan Hall, Charlottesville, VA). IFNτ can be obtained from a number of sources including cows, sheep, ox, and humans.

An *interferon-*τ *polypeptide* is a polypeptide having between about 15 and 172 amino acids derived from an interferon-τ amino acid coding sequence, where said 15 to 172 amino acids are contiguous in native interferon-τ. Such 15-172 amino acid regions can also be assembled into polypeptides where two or more such interferon-τ regions are joined that are normally discontinuous in the native protein.

*Treating* a disease refers to administering a therapeutic substance effective to reduce the symptoms of the disease and/or lessen the severity of the disease.

### II. Overview of Invention

Experiments performed in support of the present invention indicate that IFNτ is effective at preventing the development of experimental allergic encephalomyelitis (EAE; Zamvil and Steinman, 1990), an animal model of antigen-induced autoimmunity that has been widely studied to gain insight into multiple sclerosis (MS). IFNτ is at least as effective in these experiments as IFNβ, which has recently been approved by the FDA for the treatment of MS. The experiments further show that IFNτ has a lower toxicity than IFNβ, and that IFNτ-treated mice do not develop leukopenia, an undesired side effect associated with IFNβ treatment.

In addition, experiments performed in support of the present invention have demonstrated that orally-administered IFNτ is nearly as effective as injected IFNτ at treating EAE, but results in significantly lower anti-IFNτ antibody titers in the treated individuals. This unexpected advantage results in a decreased chance of side-effects due to a host immune response against IFNτ.

It has recently been shown that superantigens can include relapses in EAE, similar to those that occur "spontaneously" in MS patients. Additional experiments performed in support of the present invention show that IFNτ blocks superantigen reactivation of EAE, and that the inhibitory effect of IFNτ on induction of EAE and reactivation by superantigen involves suppression of myelin basic protein (MBP) and superantigen activation of T cells as well as suppressed induction of destructive cytokines such as tumor necrosis factor. Taken together, these results indicate that IFNτ, both injected and orally-administered, may be highly effective in treatment of autoimmune diseases, such as MS, with lower toxicity and fewer side effects than are associated with IFNβ.

### III. Interferon-τ.

The first IFNτ to be identified was ovine IFNτ (OvIFNτ). Several isoforms of the 18-19 kDa protein were identified in conceptus (the embryo and surrounding membranes) homogenates (Martal, *et al.,* 1979). Subsequently, a low molecular weight protein released into conceptus culture medium was purified and shown to be both heat labile and susceptible to proteases (Godkin, *et al.,* 1982). OvIFNτ was originally called ovine trophoblast protein-one (oTP-1) because it was the primary secretory protein initially produced by trophectoderm of the sheep conceptus during the critical period of maternal recognition in sheep. Subsequent experiments have determined that OvIFNτ is a pregnancy recognition hormone essential for establishment of the physiological response to pregnancy in ruminants, such as sheep and cows (Bazer and Johnson, 1991).

IFNτs with similar characteristics and activities have been isolated from other ruminant species including cows and goats (Bartol, *et al*., 1985; and Gnatek, *et al*., 1989). Antisera to all the IFNτs cross-react. This is not unexpected since the species specific forms of IFNτ are more closely homologous to each other than to the IFNsα from the identical species (Roberts, *et al*., 1992).

The cow protein (BOIFNτ; Helmer, *et al*., 1987; Imakawa, *et al*., 1989) has similar functions to OvIFNτ in maternal recognition of pregnancy. Further, it shares a high degree of amino acid and nucleotide sequence homology with OvIFNτ. The nucleic acid sequence homology between OvIFNτ and BoIFNτ is 76.3 % for the 5' non-coding region, 89.7% for the coding region, and 91.9% for the 3' non-coding region. The amino acid sequence homology is 80.4%.

An IFNτ cDNA obtained by probing a sheep blastocyst library with a synthetic oligonucleotide representing the N-terminal amino acid sequence (Imakawa, *et al*., 1987) has a predicted amino acid sequence that is 45-55 % homologous with IFNsα from human, mouse, rat and pig and 70% homologous with bovine IFNαII, now referred to as IFNΩ. Several cDNA sequences have been reported which may represent different isoforms (Stewart, *et al*., 1989; Klemann, *et al*., 1990; and Charlier, M., *et al*., 1991). All are approximately 1 kb with a 585 base open reading frame that codes for a 23 amino acid leader sequence and a 172 amino acid mature protein. The predicted structure of IFNτ as a four helical bundle with the amino and carboxyl-termini in apposition further supports its classification as a type I IFN (Jarpe, *et al*., 1994).

**TABLE 1**

| OVERVIEW OF THE INTERFERONS | | | | |
|---|---|---|---|---|
| **Aspects** | **Type I** | | | **Type II** |
| Types | α & ω | β | τ | γ |
| Produced by: | leukocyte | fibroblast | trophoblast | lymphocyte |
| Effects: | | | | |
| Antiviral | + | + | + | + |
| Antiproliferative | + | + | + | + |
| Pregnancy Signally | - | - | + | - |

While IFNτ displays many of the activities classically associated with type I IFNs (see Table 1, above), considerable differences exist between it and the other type I IFNs. The most prominent difference is its role in pregnancy, detailed above. Also different is viral induction. All type I IFNs, except IFNτ, are induced readily by virus and dsRNA (Roberts, *et al*., 1992). Induced IFNα and IFNβ expression is transient, lasting approximately a few hours. In contrast, IFNτ synthesis, once induced, is maintained over a period of days (Godkin, *et al*., 1982). On a per-cell basis, 300-fold more IFNτ is produced than other type I IFNs (Cross and Roberts, 1991).

Other differences may exist in the regulatory regions of the IFNτ gene. For example, transfection of the human trophoblast cell line JAR with the gene for bovine IFNτ resulted in antiviral activity while transfection with the bovine IFNΩ gene did not. This implies unique transacting factors involved in IFNτ gene expression. Consistent with this is the observation that while the proximal promoter region (from 126 to the transcriptional start site) of IFNτ is highly homologous to that of IFNα and IFNβ; the region from -126 to -450 is not homologous and enhances only IFNτ expression (Cross and Roberts, 1991). Thus, different regulatory factors appear to be involved in IFNτ expression as compared with the other type I IFNs.

IFNτ expression may also differ between species. For example, although IFNτ expression is restricted to a particular stage (primarily days 13-21) of conceptus development in ruminants (Godkin, *et al*., 1982), preliminary studies suggest that the human form of IFNτ is constitutively expressed throughout pregnancy (Whaley, *et al*., 1994).

### A. Isolation of IFNτ

OvIFNτ protein may be isolated from conceptuses collected from pregnant sheep and cultured *in vitro* in a modified Minimum Essential Medium (MEM) as described by Godkin, *et al.,* (1982) and Vallet, *et al.,* (1987). The IFNτ may be purified from the conceptus cultures by ion exchange chromatography and gel filtration. The homogeneity of isolated IFNτ may be assessed by sodium dodecyl sulfate polyacrylamide gel electrophoresis (SDS-PAGE; Maniatis, *et al*., 1982; Ausubel, *et al*., 1988), and determination of protein concentration in purified IFNτ samples may be performed using the bicinchoninic (BCA) assay (Pierce Chemical Co., Rockford, IL; Smith, *et al*., 1985).

### B. Recombinant Production of IFNτ

Recombinant IFNτ protein may be produced from any selected IFNτ polynucleotide fragment using a suitable expression system, such as bacterial or yeast cells. The isolation of IFNτ nucleotide and polypeptide sequences is described in Bazer, *et al.* (1994). For example, Bazer, *et al*., describe the identification and isolation of a human IFNτ gene. A synthetic nucleotide sequence encoding a mature human interferon-τ (HuIFNτ) protein is presented herein as SEQ ID NO:3. SEQ ID NO:4 is the corresponding amino acid sequence for a mature HuIFNτ1 protein. SEQ ID NO:5 is the nucleotide sequence, excluding leader sequence, of genomic DNA clone HuIFNτ3, a natural HuIFNτ gene, and SEQ ID NO:6 is the predicted amino acid sequence of a mature human IFNτ protein encoded by the sequence represented as SEQ ID NO:5.

To make an IFNτ expression vector, an IFNτ coding sequence *(e.g,* SEQ ID NO:1) is placed in an expression vector, *e.g.*, a bacterial expression vector, and expressed according to standard methods. Examples of suitable vectors include lambda gt11 (Promega, Madison WI); pGEX (Smith, *et al*., 1985); pGEMEX (Promega); and pBS (Stratagene, La Jolla CA) vectors. Other bacterial expression vectors containing suitable promoters, such as the T7 RNA polymerase promoter or the tac promoter, may also be used. Cloning of the OvIFNτ synthetic polynucleotide into a modified pIN III omp-A expression vector is described in the Materials and Methods.

For the experiments described herein, the OvIFNτ coding sequence present in SEQ ID NO:1 was cloned into a vector, suitable for transformation of yeast cells, containing the methanol-regulated alcohol oxidase (AOX) promoter and a Pho1 signal sequence. The vector was used to transform *P. pastoris* host cells and transformed cells were used to express the protein according to the manufacturer's instructions (Invitrogen, San Diego, CA).

Other yeast vectors suitable for expressing IFNτ for use with methods of the present invention include 2 micron plasmid vectors (Ludwig, *et al*., 1993), yeast integrating plasmids (YIps; *e.g.,* Shaw, *et al*., 1988), YEP vectors (Shen, *et al*., 1986), yeast centromere plasmids (YCps; *e.g*., Ernst, 1986), and other vectors with regulatable expression (Hilzeman, *et al*., 1988; Rutter, *et al*., 1988; Oeda, *et al*., 1988). Preferably, the vectors include an expression cassette containing an effective yeast promoter, such as the MFα1 promoter (Ernst, 1986; Bayne, *et al*., 1988, GADPH promoter (glyceraldehyde-3-phosphate-dehydrogenase; Wu, *et al*., 1991) or the galactose-inducible GAL10 promoter (Ludwig, *et al*., 1993; Feher, *et al*., 1989; Shen, *et al*., 1986). The yeast transformation host is typically *Saccharomyces cerevisiae*, however, as illustrated above, other yeast suitable for transformation can be used as well *(e.g., Schizosaccharomyces pombe*, *Pichia pastoris* and the like).

Further, a DNA encoding an IFNτ polypeptide can be cloned into any number of commercially available vectors to generate expression of the polypeptide in the appropriate host system. These systems include the above described bacterial and yeast expression systems as well as the following: baculovirus expression (Reilly, *et al*., 1992; Beames, *et al*., 1991; Clontech, Palo Alto CA); plant cell expression, transgenic plant expression *(e.g.,* Gelvin and Schilperoot), and expression in mammalian cells (Clontech, Palo Alto CA; Gibco-BRL, Gaithersburg MD). The recombinant polypeptides can be expressed as fusion proteins or as native proteins. A number of features can be engineered into the expression vectors, such as leader sequences which promote the secretion of the expressed sequences into culture medium. The recombinantly produced polypeptides are typically isolated from lysed cells or culture media. Purification can be carried out by methods known in the art including salt fractionation, ion exchange chromatography, and affinity chromatography. Immunoaffinity chromatography can be employed, as described above, using antibodies generated based on the IFNτ polypeptides.

In addition to recombinant methods, IFNτ proteins or polypeptides can be isolated from selected cells by affinity-based methods, such as by using appropriate antibodies. Further, IFNτ peptides may be chemically synthesized using methods known to those skilled in the art.

### C. IFNτ Lacks Toxicity

Type I IFNs (IFNα and IFNβ), as well as type II (IFNγ), exhibit significant cytotoxicity (Degre, 1974; Fent and Zbinden, 1987). Detrimental toxic effects exerted by these IFNs have been observed during clinical trials and patient treatment, and include flulike symptoms such as fever, chills and lethargy, tachycardia, nausea, weight loss, leukopenia, and neutropenia (Degre, 1974; Fent and Zbinden, 1987).

Experiments performed in support of the present invention and detailed in Example 1, below, suggest that IFNτ has significantly lower cytotoxicity than the IFNs listed above. Cytotoxicity was assessed *in vivo* (Example 1A) using white blood cell counts (WBC), lymphocytes percentages and total body weights of New Zealand White (NZW) mice injected with the various IFNs. The results are presented in Table 3 and summarized in Table 2a. Twelve hours after injection with 10⁵ U of murine interferon-alpha (MuIFNα), shown previously to induce a higher degree of toxicity than IFNβ, the mice exhibited decreased white blood cell counts, lymphopenia and substantial weight loss. None of these toxicity-related effects were observed in OvIFNτ-injected animals. The concentrations of OvIFNτ used in the toxicity studies were the same as those shown to be effective at preventing EAE (detailed in Example 2, below).

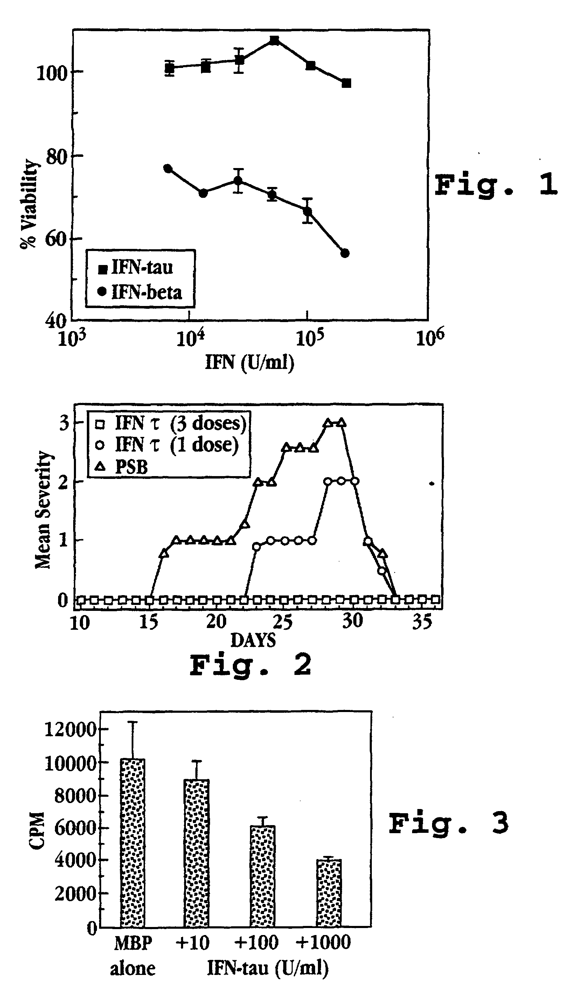

Cytotoxicity was also assessed *in vitro* (Example 1B). Viability of L929 cells exposed IFNτ at concentrations as high as 200,000 U/ml remained near control levels, while IFNβ showed toxic effects at concentrations as low as 7,000 U/ml (Figure 1). IFNτ was also found to lack toxicity when tested in a panel of tumorigenic cell lines, although it did inhibit cell replication. The results of these and additional studies, comparing the toxicity of IFNτ with the toxicities of IFNβ and IFNα in animal models as well as tissue culture (Bazer and Johnson, 1991; Johnson, *et al.,* 1994; Bazer, *et al.,* 1989; and Soos and Johnson, 1995), are summarized in Table 2a, below.

**TABLE 2A**

| PARAMETERS DEMONSTRATING THE LACK OF TOXICITY BY IFNτ BUT NOT IFNs α AND β | | | |
|---|---|---|---|
| **Toxicity** | | | |
| ***In vitro* (cell viability)** | **IFNτ** | **IFNα** | **IFNβ** |
| Mouse L929 (50,000-200,000 U/ml of IFN) | - | + | + |
| Bovine MDBK (50,000 U/ml of IFN) | - | + | ND |
| Human WISH (50.000 U/ml of IFN) | - | + | ND |
| Human Peripheral Lymphocytes (50,000 U/ml of IFN) | - | + | + |
| HIV Infected Human Peripheral Lymphocytes (50,000-500,000 U/ml of IFN) | - | + | ND |
| *In vivo* (NZW Mice) | IFNτ | IFNα | IFNβ |
| White Blood Cell Count | - | + | + |
| Lymphocyte Depression | - | + | + |
| Weight Measurement | - | + | ± |
| Plus and minus signs indicate toxicity or lack thereof induced by treatment with the various type I IFNs. For *in vivo* studies, 10⁵ U were administered per injection and cell counts and weights were evaluated at either 12 or 24 hours after injection. ND = not determined. | | | |

MDBK cells cultured in the presence of IFNs exhibited reduced viability when cultured in the presence of IFNα (50,000 U/ml), but not when cultured in the presence of IFNτ (Pontzer, *et al.,* 1991). Similar results were obtained with the human WISH cell line. Comparisons of toxicity (or lack thereof) induced by IFNτ and other IFNs have been made using human peripheral mononuclear cells (HPMC) and HIV-infected HPMC. IFNτ did not exhibit toxic effects on cultured HPMC while both IFNα and IFNβ reduced cell viability at 50,000 U/ml (Soos and Johnson, 1995). Human lymphocytes infected with HIV-1 and feline lymphocytes infected with HIV also did not exhibit reduced viability in the presence of IFNτ (Bazer, *et al*., 1989). These findings indicate that the lack of toxicity of IFNτ inferred from observations using immortalized cell lines also applies to human peripheral blood. The results summarized in Table 2a demonstrate that injected IFNτ appears to have little or no toxicity, when tested both in *vitro* and *in vivo*, as compared with injected IFNα, IFNβ and IFNγ.

Additional experiments performed in support of the present invention compared the toxicity, measured by lymphocyte depression in peripheral blood, of orally-administered and injected OvIFNτ with that of orally-administered and injected IFNs α and β. Blood was obtained from the tail and white blood cells (WBC) counts were enumerated using a hemocytometer. Differential WBC counts were performed on Wright-Giemsa-stained blood smears.

The results are shown in Tables 2b, 2c and 2d, below. Significant levels of toxicity were detected in mice fed either IFN α and β while no significant lymphocyte depression was detected in mice fed 10⁵, 2x10⁵ or 5x10⁵ U of OvIFNτ or PBS alone. These data suggest that orally-administered OvIFNτ (like injected OvIFNτ) has significantly-reduced toxicity with respect to other type I IFNs.

### Tables 2b-2d

### Comparison of IFNs τ,β and α for Toxicity After Oral Feeding

**Table 2b**

| IFN (DOSE) | CELL COUNT (CELL No. × 10³) | |
|---|---|---|
| | BEFORE ORAL FEEDING | |
| | TOTAL WBC | LYMPHOCYTES |
| PBS | 7.0 ± 1.4 | 6.1 ± 1.2 |
| τ(10⁵) | 7.5 ± 0,7 | 6.4 ± 0.6 |
| τ(2×10⁵) | 6.5 ± 0.7 | 5.3 ± 0.6 |
| τ(5×10⁵) | 7.5 ± 0.7 | 6.5 ± 0.6 |
| **β**(10⁵) | 7.0 ± 0.7 | 5.9 ± 1.2 |
| β(2×10⁵) | 7.5 ± 2.1 | 6.5 ± 1.8 |
| α(10⁵) | 7.5 ± 0.7 | 6.6 ± 0.6 |

**Table 2c**

| IFN (DOSE) | CELL COUNT (CELL No. × 10³) | | |
|---|---|---|---|
| | 18 H AFTER ORAL FEEDING | | |
| | TOTAL WBC | LYMPHOCYTES | % LYMPHOCYTE DEPRESSION |
| PBS | -- | -- | -- |
| τ(10⁵) | 7.0 ± 1.4 | 6.0 ± 1.3 | 6.2 |
| τ(2×10⁵) | 7.0 ± 2.8 | 5.9 ± 2.4 | 0 |
| τ(5×10⁵) | 7.5 ± 2.1 | 6.3 ± 1.8 | 3.1 |
| β(10⁵) | 6.5 ± 0.7 | 5.1 ± 0.6 | 13.6 |
| β(2×10⁵) | 6.5 ± 0.7 | 4.1 ± 0.4^{†} | 37.0 |
| α(10⁵) | 6.5 ± 2.1 | 4.7 ± 1.6 | 28.8 |

| | | | |
|---|---|---|---|
| ^{†}p < 0.05 | | | |

**Table 2d**

| IFN (DOSE) | CELL COUNT (CELL No. × 10³) | | |
|---|---|---|---|
| | 24 H AFTER ORAL FEEDING | | |
| | TOTAL WBC | LYMPHOCYTES | % LYMPHOCYTE DEPRESSION |
| PBS | 7.5 ± 0.7 | 6.4 ± 0.6 | 0 |
| τ(10⁵) | 8.0 ± 2.8 | 6.9 ± 2.4 | 0 |
| τ(2×10⁵) | 7.0 ± 1.4 | 6.0 ± 1.1 | 0 |
| τ(5×10⁵) | 8.0 ± 4.2 | 7.0 ± 3.6 | 0 |
| β(10⁵) | 6.5 ± 3.5 | 5.1 ± 2.8 | 13.6 |
| β(2×10⁵) | 6.5 ± 0.7 | 4.0 ± 0.4^{†} | 38.5 |
| α(10⁵) | 7.0 ± 0 | 5.0 ± 0^{‡} | 24.2 |

| | | | |
|---|---|---|---|
| ^{†}p<0.05 | | | |
| ^{‡}p<0.03 | | | |

### IV. IFNτ as a Treatment for Autoimmune Disorders

Compositions and methods of the present invention may be used to therapeutically treat and thereby alleviate a variety of immune system-related disorders characterized by hyper- or hypo-active immune system function. Such disorders include hyperallergenicity and autoimmune disorders, such as multiple sclerosis, type I (insulin dependent) diabetes mellitus, lupus erythematosus, amyotrophic lateral sclerosis, Crohn's disease, rheumatoid arthritis, stomatitis, asthma, allergies, psoriasis and the like.

### A. IFNτ treatment in EAE, an Animal Model for Multiple Sclerosis

1. OvIFNτ Inhibits Development of EAE, an Animal Model for Multiple Sclerosis. The efficacy of IFNτ in treating autoimmune disorders may be evaluated in rodents with experimental allergic encephalomyelitis (EAE), an animal model of antigen-induced autoimmunity that is widely studied to gain insight into human multiple sclerosis (MS). EAE is an autoimmune demyelinating disease induced by immunizing susceptible mouse, rat or guinea pig strains with myelin basic protein (MBP) or with encephalitogenic peptide fragments. Genetic susceptibility in the model animal strains is based in part on the capacity of encephalitogenic peptides to bind to particular class II major histocompatibility complex (MHC-II) molecules (Fritz, *et al*., 1983; Wraith, *et al*., 1989). In particular, mice having the H-2^{u} haplotype are susceptible to EAE. Susceptible mouse strains include PL/J mice (Klein, *et al*., 1983), (PL/J × SJL)F₁ mice (Zamvil, *et al*., 1990; Wraith, *et al*.), B10.PL mice (Figuero, *et al*., 1982), NZW mice (Kotzin, *et al*., 1987), and (NZB × NZW)F1 (Kotzin, *et al*.) mice.
   Gamma-interferon (IFNγ) and beta-interferon (IFNβ) have been demonstrated to be effective in treating multiple sclerosis (Johnson, *et al*., 1994; IFNβ Multiple Sclerosis Study Group, 1993). In fact, IFNβ has been approved by the FDA as a therapeutic for multiple sclerosis. Although β-IFN is effective against MS, it has relatively high toxicity, and as a result, has a variety of undesirable side effects. As described above, however, IFNτ has significantly lower toxicity that other interferons and may therefore exhibit fewer undesirable side effects.
   In experiments performed in support of the present invention and detailed in Example 2, IFN-τ was tested for its ability to prevent the induction of EAE. EAE was induced in New Zealand White (NZW) mice by immunization with bovine myelin basic protein (bMBP). The mice were injected intraperitoneally (i.p.) with either a single dose of recombinant ovine IFN-tau (OvIFNτ) or murine IFN-beta (MuIFN-β) on the day of, or 3 doses of OvIFN-τ or MuIFN-β 48 hours before, on the day of and 48 hours after immunization with MBP.
   The results of the experiments are summarized in Table 4. A time course of the mean severity of EAE is presented in Figure 2. Symbols are as follows: Δ - control animal; ⊕ - single dose of OvIFNτ; □ - 3 doses of OvIFNτ.
   All of the animals injected (both sham-injected and IFN-injected) on the day of the immunization developed EAE, but the severity was reduced, and the mean day of onset was delayed in both the OvIFNτ (23.8 ± 0.5 days) and MuIFN-β (23.5 ± 0.6 days) treated animals relative to control animals (16.2 ± 0.8 days).
   The results obtained using the 3-dose protocol are more striking. Seven of the nine control animals developed EAE an average of 15.2 days following immunization. In contrast, none of nine animals treated with OvIFNτ developed the disease, and one of nine animals treated with MuIFN-β succumbed to EAE (22 days after immunization).
   The data demonstrate that IFNτ is an effective immunotherapy for the prevention of EAE, and is as effective a treatment in this model of autoimmune disease as MuIFNβ. Taken together with the lower toxicity of IFNτ relative to IFNβ, the data suggest that treatment of individuals having an autoimmune disorder (such as multiple sclerosis) with IFNτ may be preferable and more effective than treatment with IFNβ.
2. OvIFNτ Inhibits T-Cell Proliferation. The effects of IFNτ on proliferation of spleen cells from MBP-immunized NZW mice stimulated with MBP *in vitro* were assessed. The results are shown in Figure 3. Proliferation in response to MBP was vigorous and could be reduced by IFNτ in a dose-dependent manner, indicating that IFNτ has antiproliferative activity against T cells specific for the autoantigen, MBP. These results are consistent with the observation that IFNτ inhibits or eliminates symptoms of MBP-induced EAE, since inhibition of such T-cells would be expected to reduce the severity of the autoimmune response.
3. OvIFNτ Inhibits Superantigen Reactivation of EAE. The symptomology of MS can often be observed to occur in a relapsing-remitting manner. This form of MS consists of presentation with clinical symptoms of MS followed by periods of remission. How relapses and exacerbations occur and what causes the reactivation of autoimmune disease has been a topic of much speculation. It has been suggested that environmental influences may contribute to or even be responsible for exacerbations of autoimmune disease. Such influences potentially include exposure to infectious agents as well as factors possessing immunostimulatory activity. One class of proteins which are ubiquitous in our environment are the microbial superantigens.
   Microbial superantigens are toxins produced by a variety of bacteria, viruses, and other organisms such as mycoplasma that possess extremely potent immunostimulatory activity (Langford, *et al*., 1978; Carlsson and Sjogren, 1985; and Johnson and Magazine, 1988). They are responsible for a number of maladies including food poisoning and toxic shock syndrome (Bergdoll, *et al*., 1981). Such powerful immunostimulation by superantigens is based on their ability to engage major histocompatibility complex class II molecules and then, as a binary complex, bind to the T cell receptor in a β-chain variable region (Vβ) -specific manner (Johnson, *et al*., 1991; Janeway, *et al*., 1989; White, *et al*., 1989; Carlsson, *et al*., 1988; and Fleischer and Schrezenmeier, 1988). This binding triggers T cell activation leading to proliferation of as much as 20% of a T cell repertoire (Johnson, *et al*., 1991).
   Superantigen-induced T cell proliferation is accompanied by massive amounts of cytokine production including interleukin 2 (IL2), IFNγ, and tumor necrosis factor alpha (TNFα). Of the cytokines whose production is induced by superantigen stimulation, IFNγ and TNFα have been implicated as mediators of autoimmune pathogenesis. IFNγ has been shown to cause exacerbations of MS in clinical trials (Panitch, *et al*., 1987a; Panitch, *et al.,* 1987b). Production of TNFα has been shown to be a requirement for the encephalitogenicity of certain T cell lines used to adoptively transfer EAE (Powell, *et al.,* 1990) as well as causing myelin producing oligodendrocyte death in *vitro* (Selmaj and Raine, 1988).
   Experiment performed in support of the present invention that *Staphylococcus* Enterotoxin B (SEB) -induced cytokine production is also altered by IFNτ. Spleen cells from MBP-immunized mice were stimulated with SEB *in vitro* in the presence or absence of IFNτ, and supernatants were examined for TNFα and IFNγ production. Addition of IFNτ to cultures stimulated with SEB significantly reduced production of both TNFα and IFNγ. In view of the above, these results are consistent with the ability of IFNτ to reduce the severity of EAE, and suggest that IFNτ may reduce exacerbations of MS.
   Exacerbation evidenced as a clinical relapse of EAE was first demonstrated by the administration of a microbial superantigen. In the PL/J strain, acute episodes of EAE usually resolve and clinical relapses have been shown not to occur (Fritz, et *al*., 1983). After resolution of all clinical signs of EAE induced by immunization with MBP, administration of either of the *Staphylococcus aureus* enterotoxin (SE) superantigens, SEB or *Staphylococcus* Enterotoxin A (SEA), was shown to cause reactivation of disease (Schiffenbauer, *et al*., 1993). Multiple episodes of disease exacerbation over a four-month period were also shown in which EAE could be reactivated and resolved based on multiple injections of SEB (Schiffenbauer, et *al*., 1993). Reactivation of EAE by SEB has also been shown to occur in other susceptible strains including NZW. SEB can also reactivate disease when an acetylated amino terminal peptide of MBP is employed as the immunogen (Brocke, *et al*., 1993).
   In addition to reactivation of EAE, SEB can also prevent EAE when administered prior to immunization with MBP (Soos, *et al.,* 1993; and Kalman, *et al.,* 1993). Anergy and/or deletion of the Vβ8⁺ T cell subset which is responsible for the initial induction of EAE appears to be the mechanism for this protection. Targeting of a Vβ specific T cell population does not, however, provide absolute protection from development of EAE. When mice protected from development of EAE by SEB pre-treatment are exposed to SEA (which has a different Vβ T cell specificity from SEB), induction of EAE does occur. This SEA-induced EAE is characterized by severe paralysis and accelerated onset of clinical symptoms. Thus, the effects of microbial superantigens introduce a profound complexity to autoimmune disease models such as EAE, akin to the complexity of the pathogenesis observed in MS.
   The effect of OvIFNτ treatment on exacerbations of EAE induced by superantigen is evaluated on NZW mice in Example 4. The studies have also been carried out on PL/J mice. Treatment with OvIFNτ when administered in 3 doses of 10⁵ U (48 hours prior to SEB injection, on the day of SEB injection and 48 hours after SEB injection) blocked EAE reactivation by superantigen. In comparison, untreated control groups exhibited superantigen reactivation of EAE consistent with previous studies (Schiffenbauer, *et al*., 1993).
   The observation that OvIFNτ can block superantigen-induced exacerbations of EAE may be a corollary to the reduction in disease exacerbations in MS patients undergoing treatment with IFNβ1b. A summary of the studies showing that OvIFNτ can prevent development and superantigen reactivation of EAE is presented in Table 5. The results demonstrate that IFNτ can also modulate the effects of environmental factors on the course of autoimmune disease, such as MS.
   Additional experiments performed in support of the present invention have further shown that a second immunization of MBP can not reactivate EAE, and that injection of superantigens can induce an initial episode of clinical disease in PL/J mice that had been immunized with MPB but did not develop EAE. The experiments further demonstrate that this induction can be blocked by treatment with IFNτ, and that IFNτ can block superantigen-induced exacerbations of EAE akin to the reduced exacerbations of disease observed in IFNβ1b treated MS patients.
4. IFNτ Inhibits Vβ-specific T-Cell Activation. The effect of IFNτ treatment of SEB-induced Vβ specific T cell expansion *in vitro* was evaluated as described in Example 5. Vβ specific T-cell FACS analysis was performed on naive, SEB-injected, or IFNτ and SEB -injected NZW mice. Analyses were performed 72 hours after the injections.

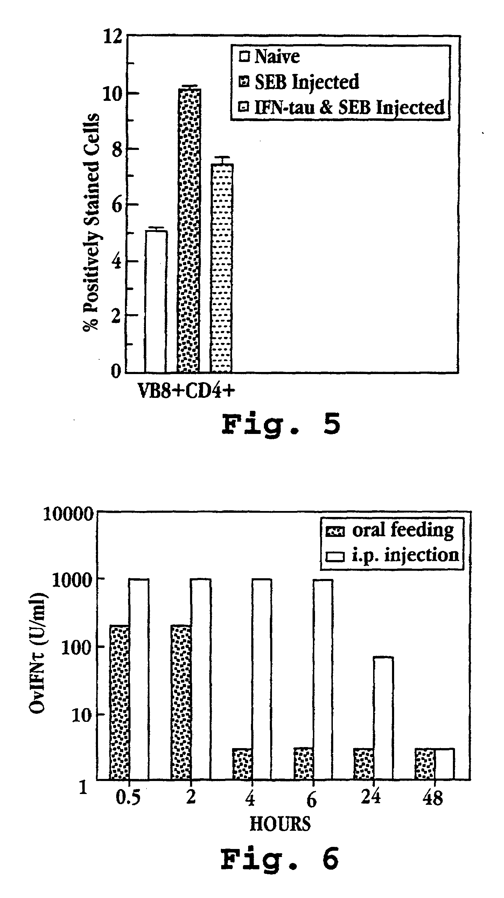

Results of exemplary experiments are shown in Figure 5. Open bars represent naive animals; closed bars represent SEB-injected animals, and crosshatched bars represent IFNτ- and SEB-injected animals. Naive NZW mice exhibited 5.1 ± 0.1 % Vβ8⁺CD4⁺ T cells, which was expanded to 10.2 ± 0.2% after injection of SEB. When an IFNτ injection preceded the SEB injection, expansion of the Vβ8⁺CD4⁺ T-subset was limited to 7.6 ± 0.2%. Partial inhibition of Vβ7⁺ and Vβ11⁺ T cells, for which SEB is also specific, was also observed.

These data indicate that treatment with IFNτ can partially inhibit SEB-induced Vβ T cell expansion *in vivo*, and further support the observation that IFNτ reduces the severity of MBP-induced EAE.

### B. Other Autoimmune Disease Models

In addition to EAE, other animal models of autoimmune disease may be used to evaluate the therapeutic effects of IFNτ. For example, certain strains of mice are particularly susceptible to murine systemic lupus erythematosus, a disease analogous to systemic lupus erythematosus in humans. In particular, the MRL-*lpr*/*lpr* lupus mouse (Singer, *et al.,* 1986) exhibits many of the same immunological characteristics of human systemic lupus erythematosus. The animals have lymphoid organ enlargement and increased T-cell proliferation, with V_{β} gene expression significantly skewed in favor of V_{β8.2/8.3} genes (Singer, *et al*.).

MRL-*lpr*/*lpr* mice may be obtained from the Jackson Laboratory (Bar Harbor, ME). The onset of disease in the MRL-*lpr*/*lpr* mice is spontaneous (at about 3 months of age), so the disease does not need to be induced as it does in the case of EAE. To evaluate the effects of IFNτ on the progression of disease, the animals are treated with injections of IFNτ *(e.g.,* as described above) at selected intervals *(e.g.,* once every two weeks) starting at a selected age (*e.g*., 6 weeks of age) for a selected duration (*e.g*., until 6 months of age).

The effects of the therapy may be evaluated in several ways, For example, the relative number of Vβ8⁺ cells in spleens and lymph nodes of treated and untreated groups of animals may be determined using FACS analysis as described above. An effective dose of IFNτ results in a significant reduction of the number of Vβ8⁺ T cells. Further, the physical symptoms of the disease (lymphoid hyperplasia, necrosis of ear, hair loss) may be quantitated (Kim, *et al*., 1991) and compared between treated and untreated groups. The animals may also be assayed for the reduction of ds-DNA-specific antibody and/or reduction in nephritis with proteinuria, for example, as described in Kim, *et al*., following treatment with IFNτ.

Another animal model of an autoimmune disorder, which may be employed to evaluate the therapeutic effects of IFNτ, is adjuvant-induced arthritis in dogs (Kaplan, *et al*., 1993).

### V. Effectiveness of Orally-Administered IFNτ

Experiments performed in support of the present invention demonstrate that orally-administered IFNτ polypeptide compositions are comparable in efficacy to injected IFNτ compositions with respect to the treatment of diseases or disease conditions which benefit from treatment with IFNτ, such as autoimmune diseases (*e.g*., multiple sclerosis).

As discussed below, not only was orally-administered IFNτ effective at treating a disease benefiting from IFNτ treatment (EAE), but the oral route of administration resulted in unexpected advantages relative to treatment with injected IFNτ compositions. For example, orally-administered IFNτ resulted in a significantly lower level of anti-IFNτ antibodies in the serum of treated individuals (see Example 12). This is beneficial because the orally-administered IFNτ is therefore less likely to be rendered ineffective by a host immune response *(i.e.,* desensitization to the treatment and/or dose level is significantly decreased), and the individual receiving the treatment is less likely to suffer adverse side effects as a result of such an immune response.

Results of experiments demonstrating these and related findings are presented below.

### A. Orally-Administered IFNτ Inhibits Development of EAE

In experiments detailed in Example 6, orally-administered and injected IFN-τ was tested for its ability to prevent the induction of EAE. EAE was induced in New Zealand White (NZW) mice by immunization with bovine myelin basic protein (bMBP). Recipient NZW mice received OvIFNτ by either i.p. injection or oral feeding 48 hours prior to, on the day of, and 48 hours after immunization with bovine myelin basic protein (bMBP) for induction of experimental allergic encephalomyelitis (EAE).

Both oral feeding and i.p. injection of OvIFNτ protected against EAE (Example 6, Table 6). All animals that received IFNτ via i.p. injection, and 7 of 9 animals that received IFNτ orally, were protected from symptoms of EAE. Furthermore, anti-OvIFNτ monoclonal antibody HL127 was effective at partially neutralizing the ability of the OvIFNτ to block EAE. These experiments demonstrate that orally-administered IFNτ is effective in treating symptoms of EAE, an animal model of multiple sclerosis.

### B. OvIFNτ is Present in Sera Following Oral Administration.

To confirm that orally-administered IFNτ enters the circulation, the sera of mice that received IFNτ by i.p injection or by oral administration were tested for the presence of IFNτ using a cytopathic effect (antiviral) assay (Familetti, *et al*., 1981) as described in Example 7.

The results are shown in Fig. 6. Specific activities are expressed in antiviral units/mg protein obtained from antiviral assays using MDBK cells. OvIFNτ was detected for up to two 2 hours following oral feeding (filled bars) at levels of 200 U/ml. These data indicate that orally-administered IFNτ enters the circulation and remains in serum for about two hours after being administered.

### C. OvIFNτ Prevents Chronic Relapse of EAE

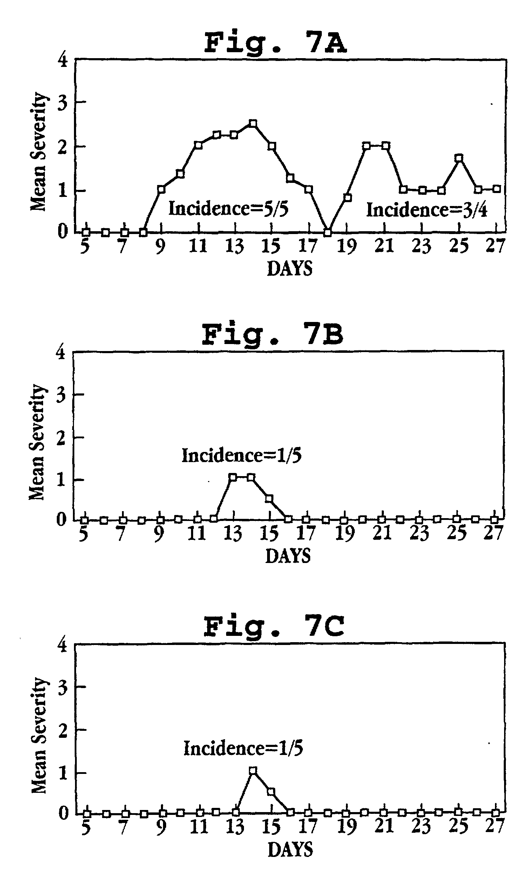

In addition to preventing the onset of symptoms associated with EAE, orally-administered OvIFNτ prevents paralysis in a chronic-relapsing model of EAE, as detailed in Example 8. Whereas 5/5 mice immunized with MBP (to induce EAE) which did not receive OvIFNτ treatment developed chronic relapsing paralysis, 4/5 animals treated with OvIFNτ (either i.p. injection or oral feeding, administered every 48 hours) were fully protected from the disease (Figs. 7B and 7C). These data further support the results described above, and indicate that oral administration of IFNτ can block the development of chronic relapsing EAE. The experiments also suggest that orally-administration of IFNτ as infrequently as once every 48 hours, over an extended period of time, is as effective as i.p. injection at treating a disease condition responsive to treatment by interferon-tau.

### D. Histological Analyses of Spinal Chord from EAE Mice following Oral Administration of IFNτ.

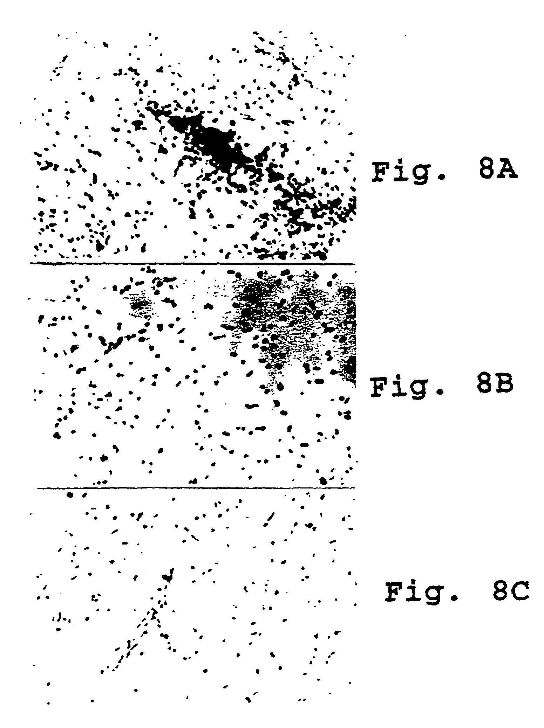

The ability of OvIFNτ to prevent EAE was also assayed by analyzing the effect of OvIFNτ treatment on cellular consequences of the disease, manifested in the central nervous system (CNS) as lymphocytic lesions in spinal cord white matter. The lesions are indicative of the extent of lymphocyte infiltration into the CNS. MBP-immunized mice were either not treated (control) or treated with OvIFNτ by oral or i.p. routes, and sections of the spinal cord lumbar region were stained and evaluated for lymphocytes as described in Example 9. Lymphocytic lesions were present in spinal cord white matter of control animals (Fig. 8A), but not in mice treated with OvIFNτ by i.p. injection (Fig. 8B) or oral feeding (Fig. 8C). These data indicate that the protective effect of IFNτ is associated with inhibition of lymphocyte infiltration of the CNS. Further, the data demonstrate that IFNτ treatment inhibits cellular manifestation of the autoimmune disease, rather than simply masking symptoms.

### E. Cessation of Treatment with OvIFNτ Results in Relapsing Paralysis.

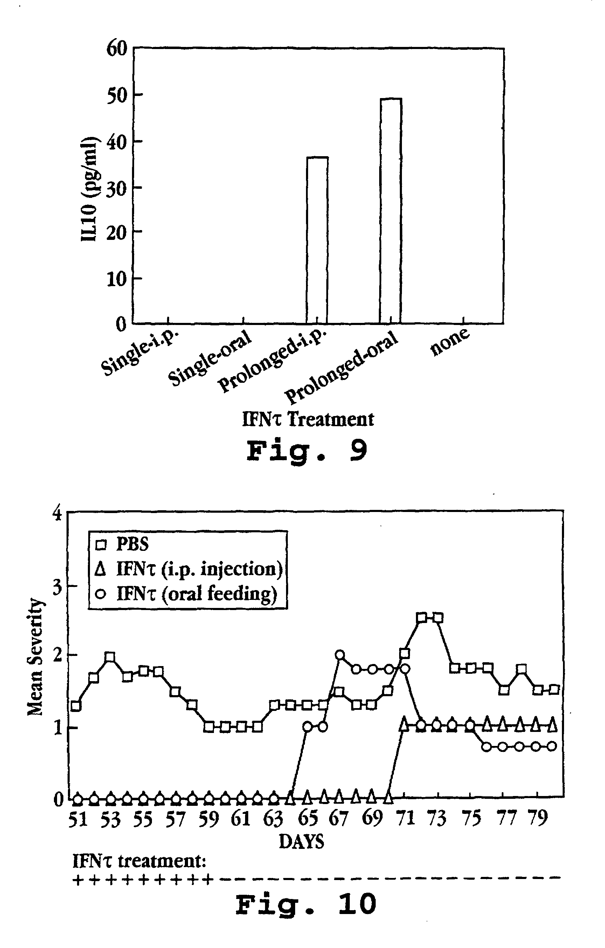

Experiments detailed in Example 11 were performed to determine the type and duration of treatment effective to prevent EAE in mice injected with MBP. The mice were protected from EAE by OvEPNτ treatment via i.p. injection or oral feeding (every 48 hours) as long as the treatment persisted (58 days in Example 11), but developed symptoms of the disease after OvIFNτ treatment was stopped (Figure 10). These results suggest that while IFNτ may not cure an autoimmune condition like EAE (*e.g*., MS), it is an effective treatment that inhibits the pathological manifestations of the condition so long as treatment is continued.

### F. Oral Administration of OvIFNτ Reduces Anti-OvIFNτ Antibody Response.

As detailed in Example 12, one advantage of orally-administered (as opposed to injected) IFNτ treatment is a reduction in the anti-IFNτ antibody titer in individuals receiving the oral treatment. After removal of OvIFNτ treatment, mice from each treatment group were bled and sera were examined for the presence of anti-OvIFNτ antibodies by ELISA. Whereas mice receiving IFNτ by i.p. injection exhibited elevated levels of anti-IFNτ antibodies, animals receiving IFNτ by oral feeding exhibited much lower anti-IFNτ antibody titers (typically 3 to 5 -fold lower). As expected mice which received no OvIFNτ treatment displayed no anti-OvIFNτ antibodies.

The sera were also examined for their ability to neutralize OvIFNτ antiviral activity on the MDBK cell line. None of the sera from either i.p. injected or orally fed mice possessed neutralizing activity (Table 7). These results suggest that oral feeding of OvIFNτ largely circumvents an antibody response directed against the OvIFNτ protein. Such a reduced antibody response in orally-treated subjects reduces the chance of undesirable immune system-related side effects of IFNτ treatment.

### VI. Applications

### A. IFNτ as a Treatment for Immune System Disorders

Diseases which may be treated using methods of the present invention include autoimmune, inflammatory, proliferative and hyperproliferative diseases, as well as cutaneous manifestations of immunologically mediated diseases. In particular, methods of the present invention are advantageous for treating conditions relating to immune system hypersensitivity. There are four types of immune system hypersensitivity (Clayman). Type I, or immediate/anaphylactic hypersensitivity, is due to mast cell degranulation in response to an allergen (*e.g.*, pollen), and includes asthma, allergic rhinitis (hay fever), urticaria (hives), anaphylactic shock, and other illnesses of an allergic nature. Type II, or autoimmune hypersensitivity, is due to antibodies that are directed against perceived "antigens" on the body's own cells. Type III hypersensitivity is due to the formation of antigen/antibody immune complexes which lodge in various tissues and activate further immune responses, and is responsible for conditions such as serum sickness, allergic alveolitis, and the large swellings that sometimes form after booster vaccinations. Type IV hypersensitivity is due to the release of lymphokines from sensitized T-cells, which results in an inflammatory reaction. Examples include contact dermatitis, the rash of measles, and "allergic" reactions to certain drugs.

The mechanisms by which certain conditions may result in hypersensitivity in some individuals are generally not well understood, but may involve both genetic and extrinsic factors. For example, bacteria, viruses or drugs may play a role in triggering an autoimmune response in an individual who already has a genetic predisposition to the autoimmune disorder. It has been suggested that the incidence of some types of hypersensitivity may be correlated with others. For example, it has been proposed that individuals with certain common allergies are more susceptible to autoimmune disorders.

Autoimmune disorders may be loosely grouped into those primarily restricted to specific organs or tissues and those that affect the entire body. Examples of organ-specific disorders (with the organ affected) include multiple sclerosis (myelin coating on nerve processes), type I diabetes mellitus (pancreas), Hashimotos thyroiditis (thyroid gland), pernicious anemia (stomach), Addison's disease (adrenal glands), myasthenia gravis (acetylcholine receptors at neuromuscular junction), rheumatoid arthritis (joint lining), uveitis (eye), psoriasis (skin), Guillain-Barré Syndrome (nerve cells) and Grave's disease (thyroid). Systemic autoimmune diseases include systemic lupus erythematosus and dermatomyositis.

Other examples of hypersensitivity disorders include asthma, eczema, atopical dermatitis, contact dermatitis, other eczematous dermatitides, seborrheic dermatitis, rhinitis, Lichen planus, Pemplugus, bullous Pemphigoid, Epidermolysis bullosa, uritcaris, angioedemas, vasculitides, erythemas, cutaneous eosinophilias, Alopecia areata, atherosclerosis, primary biliary cirrhosis and nephrotic syndrome. Related diseases include intestinal inflammations, such as Coeliac disease, proctitis, eosinophilia gastroenteritis, mastocytosis, inflammatory bowel disease, Chrohn's disease and ulcerative colitis, as well as food-related allergies.

Autoimmune diseases particularly amenable for treatment using the methods of the present invention include multiple sclerosis, type I (insulin dependent) diabetes mellitus, lupus erythematosus, amyotrophic lateral sclerosis, Crohn's disease, rheumatoid arthritis, stomatitis, asthma, uveitis, allergies and psoriasis.

Medicaments containing IFNτ may be used to therapeutically treat and thereby alleviate symptoms of autoimmune disorders such as those discussed above. Treatments with such medicaments are exemplified herein with respect to the treatment of EAE, an animal model for multiple sclerosis.

### B. IFNτ as Treatment for Reproductive Disorders.

Although IFNτ bears some similarity to the IFNα family based on structure and its potent antiviral properties, the IFNαs do not possess the reproductive properties associated with IFNτ. For example, recombinant human IFNα had no effect on interestrous interval compared to IFNτ, even when administered at twice the dose (Davis, *et al*., 1992).

Therefore, although IFNτ has some structural similarities to other interferons, it has very distinctive properties of its own: for example, the capability of significantly influencing the biochemical events of the estrous cycle.

The IFNτ compositions of the present invention can be used in methods of enhancing fertility and prolonging the life span of the *corpus luteum* in female mammals as generally described in Hansen, *et al*. (1991). According to the teachings herein, such methods of enhancing fertility include oral administration of a medicament containing IFNτ in a therapeutically or pharmaceutically effective amount. Further, the compositions may be similarly employed to regulate growth and development of uterine and/or fetal-placental tissues. Compositions containing human IFNτ are particularly useful for treatment of humans, since potential antigenic responses are less likely using a same-species protein.

### C. IFNτ as an Antiviral Treatment

The antiviral activity of IFNτ has broad therapeutic applications without the toxic effects that are usually associated with IFNαs. As described above, IFNτ exerts its therapeutic activity without adverse effects on the cells. The relative lack of cytotoxicity of IFNτ makes it extremely valuable as an *in vivo* therapeutic agent and sets IFNτ apart from most other known antiviral agents and all other known interferons.

Formulations or medicaments containing IFNτ can be orally-administered to inhibit viral replication. Further, the compositions can be employed in methods for affecting the immune relationship between fetus and mother, for example, in preventing transmission of maternal viruses (*e.g*., HIV) to the developing fetus. Compositions containing a human interferon-τ are particularly useful for treatment of humans, since potential antigenic responses are less likely using a homologous protein.

Examples of specific viral diseases which may be treated by orally-administered IFNτ include, but are not limited to, hepatitis A, hepatitis B, hepatitis C, non-A, non-B, non-C hepatitis, Epstein-Barr viral infection, HIV infection, herpes virus (EB, CML, herpes simplex), papilloma, poxvirus, picorna virus, adeno virus, rhino virus, HTLV I, HTLV II, and human rotavirus.

### D. IFNτ as an Antiproliferative Treatment

IFNτ exhibits potent anticellular proliferation activity. Accordingly, pharmaceutical compositions or medicaments containing IFNτ suitable for oral administration can be used to inhibit cellular growth without the negative side effects associated with other interferons which are currently known. Such compositions or formulations can be used to inhibit, prevent, or slow tumor growth.

Examples of specific cell proliferation disorders which may be treated by orally-administered IFNτ include, but are not limited to, hairy cell leukemia, Kaposi's Sarcoma, chronic myelogenous leukemia, multiple myeloma, superficial bladder cancer, skin cancer (basal cell carcinoma and malignant melanoma), renal cell carcinoma, ovarian cancer, low grade lymphocytic and cutaneous T cell lymphoma, and glioma.

Furthermore, the development of certain tumors is mediated by estrogen. Experiments performed in support of the present invention indicate that IFNτ can suppress estrogen receptor numbers. Therefore, the IFNτ-containing compositions may be particularly useful in the treatment or prevention of estrogen-dependent tumors.

### E. Veterinary Applications

In addition to the uses of the methods of the present invention detailed above, it will be appreciated that the methods may be applied to the treatment of a variety of immune system disorders suffered by domesticated and wild animals. For example, hypothyroidism in dogs typically results from a progressive destruction of the thyroid, which may be associated with Lymphocytic thyroiditis (Kemppainen and Clark, 1994). Lymphocytic thyroiditis, which resembles Hashimoto's thyroiditis in humans, is thought to be an autoimmune disorder. According to the guidance presented herein, hypothyroidism due to Lymphocytic thyroiditis in dogs may be treated with medicaments containing IFNτ as described above.

Another type of autoimmune disorder in dogs that may be alleviated by treatment with IFNτ is characterized by antinuclear antibody (ANA) positivity, pyrexia and seronegative arthritis (Day, *et al*., 1985). Immune-mediated thrombocytopenia (ITP; Kristensen, *et al*., 1994; Werner, *et al*., 1985), systemic lupus erythematosus (Kristensen, *et al*., 1994), and leukopenia and Coomb's positive hemolytic anemia (Werner, *et al*., 1985), may also be amenable to treatment using methods and compositions of the present invention.

### VII. Administration of IFNτ

### A. Pharmaceutical Compositions.

Therapeutic preparations or medicaments containing IFNτ or related polypeptides or proteins can be formulated and manufactured according to known methods for preparing pharmaceutically useful compositions (medicaments). Formulations comprising interferons or interferon-like compounds have been previously described (*e.g*., Martin, 1976). In general, the IFNτ-containing medicaments will be formulated such that an effective amount of the IFNτ is combined with a suitable carrier and/or excipient in order to facilitate effective administration of the composition. IFNτ, or related polypeptides, may be administered to a patient in any pharmaceutically acceptable dosage form, including intravenous, intramuscular, intralesional, or subcutaneous injection. Specifically, compositions and methods used for other interferon compounds can be used for the delivery of these compounds.

In the case of compositions suitable for oral administration, tablets and capsules containing IFNτ may be manufactured from IFNτ (*e.g*., lyophilized IFNτ protein) and, optionally, additives such as pharmaceutically acceptable carriers (*e.g*., lactose, corn starch, light silicic anhydride, microcrystalline cellulose, sucrose), binders (*e.g.,* alpha-form starch, methylcellulose, carboxymethylcellulose, hydroxypropylcellulose, hydroxypropylmethylcellulose, polyvinylpyrrolidone), disintegrating agents (*e.g.*, carboxymethylcellulose calcium, starch, low substituted hydroxy-propylcellulose), surfactants (*e.g.*, Tween 80, polyoxyethylene-polyoxypropylene copolymer), antioxidants (*e.g.,* L-cysteine, sodium sulfite, sodium ascorbate), lubricants (*e.g.,* magnesium stearate, talc), and the like.

Further, IFNτ polypeptides can be mixed with a solid, pulverulent or other carrier, for example lactose, saccharose, sorbitol, mannitol, starch, such as potato starch, com starch, millopectine, cellulose derivative or gelatine, and may also include lubricants. such as magnesium or calcium stearate, or polyethylene glycol waxes compressed to the formation of tablets. By using several layers of the carrier or diluent, tablets operating with slow release can be prepared.

Liquid preparations for oral administration can be made in the form of elixirs, syrups or suspensions, for example solutions containing from about 0.1 % to about 30% by weight of IFNτ, sugar and a mixture of ethanol, water, glycerol, propylene, glycol and possibly other additives of a conventional nature.

### B. Dosage

An orally-active IFNτ pharmaceutical composition is administered in a therapeutically-effective amount to an individual in need of treatment. The dose may vary considerably and is dependent on factors such as the seriousness of the disorder, the age and the weight of the patient, other medications that the patient may be taking and the like. This amount or dosage is typically determined by the attending physician. The dosage will typically be between about 1 × 10⁵ and 1 × 10⁸ units/day, preferably between about 1 × 10⁶ and 1 × 10⁷ units/day. It will be appreciated that because of its lower toxicity, IFNτ can be administered at higher doses than, for example, IFNβ. By way of comparison, patients with multiple sclerosis (MS) were treated with 10⁶ U and 8 × 10⁶ U of IFNβ. Patients receiving 8 × 10⁶ U suffered fewer relapses of disease than did patients receiving 10⁶ U. However, patients receiving the higher dose of IFNβ (8 × 10⁶ U) also exhibited more side-effects associated with IFNβ's toxicity (IFNβ Multiple Sclerosis Study Group). In view of the lower toxicity of IFNτ, these higher effective dosages could be administered without the associated toxic side-effects.

Disorders requiring a steady elevated level of IFNτ in plasma will benefit from oral administration as often as about every two to four hours or administration via injection about every 12-24 hours, while other disorders, such as MS, may be effectively treated by administering a therapeutically-effective dose at less frequent intervals, *e.g*., once every 48 hours. The rate of administration of individual doses is typically adjusted by an attending physician to enable administration of the lowest total dosage while alleviating the severity of the disease being treated.

Once improvement of a patient's condition has occurred, a maintenance dose is administered if necessary. Subsequently, the dosage or the frequency of administration, or both, may be reduced, as a function of the symptoms, to a level at which the improved condition is retained.

Autoimmune disorders effecting the skin, such as psoriasis, can be treated intralesionally using IFNτ, wherein formulation and dose will depend on the method of administration and on the size and severity of the lesion to be treated. Preferred methods include intradermal and subcutaneous injection. Multiple injections into large lesions may be possible, and several lesions on the skin of a single patient may be treated at one time. The schedule for administration can be determined by a person skilled in the art. Formulations designed for sustained release can reduce the frequency of administration.

Regional treatment with the IFNτ polypeptides of the present invention is useful for treatment of autoimmune diseases in specific organs. Treatment can be accomplished by intraarterial infusion or intravenous injection. A catheter can be surgically or angiographically implanted to direct treatment to the affected organ. A subcutaneous portal, connected to the catheter, can be used for chronic treatment, or an implantable, refillable pump may also be employed.

Alternatively, the composition may be administered by direct injection into the affected tissue. For treating rheumatoid arthritis, for example, the composition may be administered by direct injection into the affected joint. The patient may be treated at repeated intervals of at least 24 hours, over a several week period following the onset of symptoms of the disease in the patient.

Systemic treatment is essentially equivalent for all applications. Systemic treatment using oral administration is discussed above. Multiple intravenous or subcutaneous doses are also possible, and in the case of implantable methods for treatment, formulations designed for sustained release are particularly useful. Patients may also be treated using implantable subcutaneous portals, reservoirs, or pumps. Other methods of administration include suppository and intra-vaginal. For the treatment of systemic lupus erythematosus (SLE) or MS, for example, the composition may be administered by oral or parenteral administration, such as IV administration.

### C. Combination Therapies

It will, of course, be understood that the compositions and methods of this invention may be used in combination with other therapies. For example, in view of IFNτ's relative lack of toxicity at high dosages, MS patients that do not show improvement at IFNβ1b's low dosage or could not tolerate IFNβ1b due to toxicity may benefit from subsequent or simultaneous treatment with higher dosages of IFNτ or peptides derived therefrom. In this respect, IFNβ1b may be considered a "second treatment agent". Further, development of neutralizing antibodies has been demonstrated in IFNβ1b treated patients (Weinstock-Guttman, *et al*., 1995). In cases where such neutralizing antibodies prove to impede the effectiveness of IFNβ1b, IFNτ may be an important alternative therapy, since antibody cross-reactivity is unlikely to occur, and IFNτ is unlikely to generate neutralizing antibodies (see Example 12). Orally-administered IFNτ is particularly advantageous in this respect, since it causes a significantly lower anti-IFNτ antibody response than injected IFNτ.

Another type of combination therapy enabled by the present invention is the oral administration of an antigen against which an autoimmune response is directed in combination with IFNτ. Oral administration of such an antigen can result in tolerization, reducing the severity of the autoimmune disease (for review, see, *e.g.,* Weiner, *et al*., 1994). It is contemplated that the IFNτ has a synergistic effect with the tolerization induced by the antigen, thereby alleviating the severity of the autoimmune disease. For example, MBP has been shown to suppress EAE (Lider, *et al*., 1989). According to the methods of the present invention, MBP may be administered (as a "second treatment agent") in combination with IFNτ to treat multiple sclerosis. Other examples include administration of IFNτ with collagen to treat rheumatoid arthritis, and with acetylcholine receptor polypeptides to treat myasthenia gravis.

Furthermore, IFNτ may be orally administered with known immunosuppressants, *e.g.*, steroids, to treat autoimmune diseases such a multiple sclerosis. The immunosuppressants (considered "second treatment agents") may act synergistically with IFNτ and result in a more effective treatment that could be obtained with an equivalent dose of IFNτ or the immunosuppressant alone.

Similarly, in a treatment for a cancer or viral disease, IFNτ may be administered in conjunction with, *e.g*., a therapeutically effective amount of one or more chemotherapy agents such as busulfan, 5-fluoro-uracil (5-FU), zidovudine (AZT), leucovorin, melphalan, prednisone, cyclophosphamide, dacarbazine, cisplatin, and dipyridamole.

The following examples illustrate but in no way are intended to limit the present invention.

### MATERIALS AND METHODS

### A. Buffers

### Phosphate-buffered saline (PBS)

10 × stock solution, 1 liter:
80 g NaCl
2 g KCl
11.5 g Na₂HPO4-7H₂O
2 g KH₂PO₄
Working solution, pH 7.3:
137 mM NaCI
2.7 mM KCI
4.3 mM Na₂HPO₄-7H₂O
1.4 mM KH₂PO₄

### B. General ELISA Protocol for Detection of Antibodies

Polystyrene 96 well plates Immulon II (PGC) were coated with 5 µg/mL (100 µL per well) antigen in 0.1 M carbonate/bicarbonate buffer, pH 9.5. The plates were sealed with parafilm and stored at 4°C overnight.

Following incubation, the plates were aspirated and blocked with 300 µL 10% NGS and incubated at 37°C for 1 hr. The plates were then washed 5 times with PBS 0.5% "TWEEN-20". Antisera were diluted in 0.1 M PBS, pH 7.2. The desired dilution(s) of antisera (0.1 mL) were added to each well and the plates incubated 1 hour at 37°C. The plates were then washed 5 times with PBS 0.5% "TWEEN-20".

Horseradish peroxidase (HRP) conjugated goat anti-human antiserum (Cappel, Durham, NC) was diluted 1/5,000 in PBS. 0.1 mL of this solution was added to each well. The plate was incubated 30 min at 37°C, then washed 5 times with PBS.

Sigma ABTS (substrate) was prepared just prior to addition to the plate. The reagent consists of 50 mL 0.05 M citric acid, pH 4.2, 0.078 mL 30% hydrogen peroxide solution and 15 mg ABTS. 0.1 mL of the substrate was added to each well, then incubated for 30 min at room temperature. The reaction was stopped with the addition of 0.050 mL 5% SDS (w/v). The relative absorbance is determined at 410 nm.

### C. Production of OvIFN-τ

A synthetic OvIFNτ gene was generated using standard molecular methods (Ausubel, *et al*., 1988) by ligating oligonucloetides containing contiguous portions of a DNA sequence encoding the OvIFNτ amino acid sequence (Imakawa, *et al*., 1987). The resulting IFNτ polynucleotide coding sequence spans position 16 through 531: a coding sequence of 172 amino acids.

The full length synthetic gene *Stu*I/*Sst*I fragment (540 bp) was cloned into a modified pIN III omp-A expression vector and transformed into a competent SB221 strain of *E. coli.* For expression of the IFNτ protein, cells carrying the expression vector were grown in L-broth containing ampicillin to an OD (550 nm) of 0.1-1, induced with IPTG (isopropyl-1-thio-b-D-galactoside) for 3 hours and harvested by centrifugation. Soluble recombinant IFNτ was liberated from the cells by sonication or osmotic fractionation.

For expression in yeast, the IFNτ gene was amplified using polymerase chain reaction (PCR; Mullis, 1987; Mullis, *et al*., 1987) with PCR primers containing *Stul* and *Sacl* restriction sites at the 5' and 3' ends, respectively. The amplified fragments were digested with *Stu*I and *Sac*II and ligated into the *Sac*II and *Sma*I sites of "pBLUESCRIPT+(KS)", generating pBSY-IFNτ. Plasmid pBSY-IFNτ was digested with *Sac*II and *Eco*RV and the fragment containing the synthetic IFNτ gene was isolated. The yeast expression vector pBS24Ub (Sabin, *et al*., 1989; Ecker, *et al*., 1989) was digested with *Sal*I. Blunt ends were generated using T4 DNA polymerase. The vector DNA was extracted with phenol and ethanol precipitated (Sambrook, *et al*., 1989). The recovered plasmid was digested with *Sac*II, purified by agarose gel electrophoresis, and ligated to the *Sac*II-*Eco*RV fragment isolated from pBSY-IFNτ. The resulting recombinant plasmid was designated pBS24Ub-IFNτ.

The recombinant plasmid pBS24Ub-IFNτ was transformed into *E. coli.* Recombinant clones containing the IFNτ insert were isolated and identified by restriction enzyme analysis. IFNτ coding sequences were isolated form pBS24Ub-IFNτ and cloned into a *Pichia pastoris* vector containing the alcohol oxidase (AOX1) promoter (Invitrogen, San Diego, CA). The vector was then used to transform *Pichia pastoris* GS115 His host cells and protein was expressed following the manufacturer's instructions. The protein was secreted into the medium and purified by successive DEAF-cellulose and hydroxyapatite chromatography to electrophoretic homogeneity as determined by SDS-PAGE and silver staining. The purified protein had a specific activity of about 0.29 to about 0.44 × 10⁸ U/mg as measured by anti-viral activity on Madin-Darby bovine kidney (MDBK) cells.

### EXAMPLE 1

### Toxicity of IFNβ, IFNγ and IFNτ

### A. In Vivo Toxicity - Cell Counts and Weight Changes

The effects of *in vivo* treatment with IFNτ, IFNβ and IFNα (10⁵ U/injection) on total white blood cell (WBC), total lymphocyte counts and weight measurements in NZW mice were assessed as follows. Interferons (OvIFNτ, MUIFNβ, and MuIFNα) were injected intraperitoneally (i.p.) at a concentration of 10⁵ U in a total volume of 0.2 ml in PBS into groups of New Zealand White (NZW) mice (Jackson Laboratories, Bar Harbor, ME). Three to four animals were included in each group. White blood cell (WBC) counts were determined before injection and at selected timepoints thereafter (typically 12 and 24 hours) using a hemocytometer and standard techniques. Differential WBC counts were performed on Wright-Giemsa stained blood smears. Before injection, the weights of the animals ranged from 20 to 23 grams. The results are summarized in Table 3, below.

**TABLE 3**

| IN VIVO TOXICITY OF INTERFERONS AS MEASURED BY WHITE BLOOD CELL COUNTS AND PERCENT WEIGHT CHANGE | | | | | | |
|---|---|---|---|---|---|---|
| **IFN** | **Cell Count (Cell No. × 10**^{**3**}**)** | | | | **% Lymphocyte Depression** | **% Weight Change 24 Hours after Injection** |
| | **Before Injection** | | **12 hr. after Injection** | | | |
| | **Total WBC** | **Lymphocytes** | **Total WBC** | **Lymphocytes** | | |
| none | 7.3 ± 1.0 | 6.4 ± 0.7 | 8.0 ± 0.8 | 7.1 ± 0.7 | 0 | +0.5 ± 0.7 |
| τ | 6.7 ± 0.7 | 5.9 ± 0.6 | 6.7 ± 0.5 | 5.8 ± 0.4 | 1.7 | +1.3 ± 0.5 |
| β | 7.0 ± 1.4 | 6.0 ± 0.5 | 6.8 ± 0.8 | 4.1 ± 0.3 | 31.7 | -20.0 ± 1.0 |
| α | 6.0 ± 0.8 | 5.2 ± 0.7 | 4.8 ± 0.5 | 2.3 ± 0.2 | 55.8 | -8.5 ± 2.0 |

No significant differences in WBC counts, lymphocyte counts or weight change were observed between IFNτ-treated and untreated mice. In contrast, IFNβ-treated mice exhibited a 31.7% depression in lymphocyte counts 12 hours after injection, which continued for at least the next 12 hours. IFNα-treated mice exhibited a 55.8% lymphocyte depression and significant weight loss 12 hours after injection. These data indicate that, unlike IFNβ and IFNα, IFNτ lacks toxicity *in vivo* at the above concentrations as evidenced by peripheral blood cell counts and weight measurements.

### B. In Vitro Toxicity - L929 Cell Assay

The toxicity of IFN treatment was measured *in vitro* using the mouse L929 cell line. L929 cells were treated with 6000 U/ml to 200,000 U/ml of either OvIFNτ or MuIFNβ. The interferons were added at time zero and the cells were incubated for 72 hours and stained with crystal violet. The percentage of living cells was determined by measuring the absorbance at 405 nm.

Exemplary data are shown in Figure 1. Values are presented as. percent viability ± standard error in which 100 percent is equal to the viability of L929 cells treated with media alone. At 6000 U/ml. IFNβ-treated cells exhibited a 77.0 ± 0.6% viability. Viability of L929 cells decreased as the concentrations of IFNβ increased in a dose-dependent manner. In contrast, L929 cells showed no decrease in viability at any of the IFNτ concentrations tested. These data indicate that, unlike IFNβ, IFNτ lacks toxicity at high concentrations *in vitro.*

Taken together, the results summarized above demonstrate that IFNτ is essentially non-toxic at concentrations at which IFNβ induces toxicity both *in vitro* and *in vivo.*

### EXAMPLE 2

### IFNτ Inhibits Development of Experimental Allergic Encephalomyelitis

IFN-τ was tested for its ability to prevent the induction of EAE. Recipient NZW mice were injected i.p. with either a single dose of 10⁵ U/ml recombinant ovine IFN-tau (OvIFNτ) or murine IFN-beta (MuIFN-β; Lee Biomolecular, San Diego, CA) on the day of immunization with bovine myelin basic protein (bMBP) for induction of EAE or 3 doses of 10⁵ U/ml of OvIFN-τ or MuIFN-β 48 hours before, on the day of and 48 hours after immunization with MBP for induction of EAE.

For induction of EAE, 300 µg of bMBP was emulsified in complete Freund's adjuvant containing 8 mg/ml of H37Ra and injected on either side of the base of the tail. On the day of immunization and 48 hours later, 400 ng of Pertussis toxin (List Biologicals, Campbell, CA) was also injected. Mice were examined daily for signs of EAE and severity of disease was graded on the following scale: 1, loss of tail tone; 2, hind limb weakness; 3, paraparesis; 4, paraplegia; 5, moribund/death.

**Table 4**

| Effects of IFN-τ on Development of EAE | | | | |
|---|---|---|---|---|
| **Treatment** | **# of IFN Doses** | **Disease Incidence** | **Mean Day of Onset** | **Mean Severity** |
| none | 0 | 5/5 | 16.2 ± 0.8 | 3.0 ± 1.0 |
| oIFNτ | 1 | 5/5 | 23.8 ± 0.5 | 2.0 ± 1.0 |
| MuIFN-β | 1 | 4/4 | 23.5 ± 0.6 | 2.1 ± 1.6 |
| None | 0 | 7/9 | 15.3 ± 1.4 | 2.6 ± 0.8 |
| oIFNτ | 3 | 0/9 | -- | -- |
| MuIFN-β | 3 | 1/9 | 22 | 0.5 |

The results of the experiments are summarized in Table 4, above. The data are split into two sets. The first set (first three rows) corresponds to experiments where IFN was injected into experimental animals on the day of the immunization. All of the animals in this set developed EAE, but the mean day of onset was delayed in both the OvIFNτ (23.8 ± 0.5 days) and MuIFN-β (23.5 ± 0.6 days) treated animals relative to control animals (16.2 ± 0.8 days). Further, the mean severity of the disease, quantitated as described above, was reduced in both IFN-treated groups relative to controls. Like OvIFNτ, a single dose of 10⁵ U of MuIFNβ also caused a 7 day delay in the development of disease.

The results are more striking for the multiple dose protocol (rows 4-6 of Table 1), where three doses of IFN (48 hours prior, day of, and 48 hours post immunization) were administered to the experimental animals. Although seven of the nine control animals developed EAE an average of 15.2 days following immunization, none of nine animals treated with OvIFNτ developed the disease. Of the nine animals treated with MUIFN-β, one succumbed to EAE 22 days after immunization.

A time course of the mean severity from the experiments described above is presented in Figure 2. Data from control animals are indicated by (Δ), data from animals treated with a single dose of OvIFNτ are indicated by (⊕), and data from animals that received 3 doses of OvIFNτ are indicated by (□).

The data demonstrate that IFNτ is an effective immunotherapy for the prevention of EAE and is as effective a treatment as MuIFNβ in this model of autoimmune disease. Taken together with the lower toxicity of IFNτ relative to IFNβ, the data suggest that treatment of individuals having an autoimmune disorder (such as multiple sclerosis) with IFNτ may be preferable and more effective than treatment with IFNβ.

### EXAMPLE 3

### IFNτ Inhibition of T-Cell Proliferation

The effects of IFNτ on proliferation of spleen cells from MBP-immunized NZW mice stimulated with MBP *in vitro* were determined as follows. Spleen cells from NZW mice immunized with bMBP were cultured in 300 µg/ml of bMBP in the presence of [³H]thymidine and 0, 10, 100, or 1000 U/ml of OvIFNτ. Proliferation was measured by [³H]thymidine incorporation.

The results are shown in Figure 3. Data are presented as mean counts per minute (cpm) of triplicate samples. Background cpm have been subtracted from the cpm values presented. Proliferation in response to MBP was vigorous and could be reduced by IFNτ in a dose-dependent manner. 1000 U/ml IFNτ reduced proliferation to less than half of that observed in response to MBP alone.

These results demonstrate that IFNτ has antiproliferative activity against T cells specific for the autoantigen, MBP, and are consistent with the observation that IFNτ inhibits or eliminates symptoms of MBP-induced EAE.

### EXAMPLE 4

### IFNτ Prevents Superantigen Reactivation

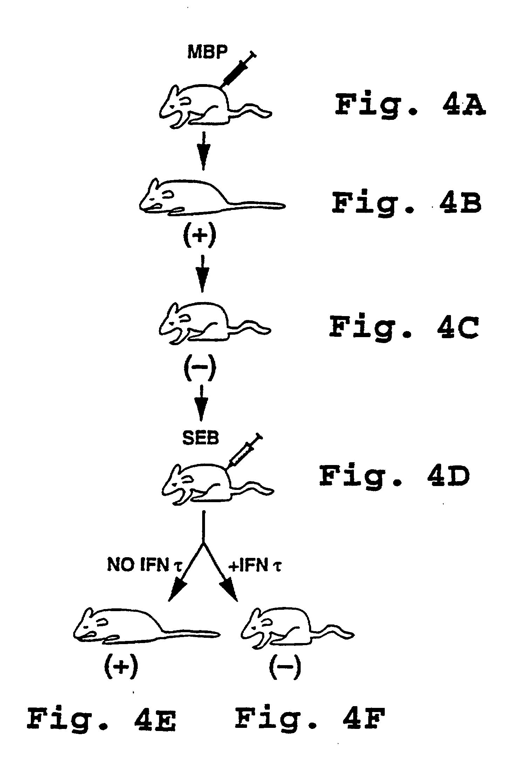

IFNτ was examined for its ability to prevent superantigen reactivation of EAE in NZW mice (Jackson Laboratory, Bar Harbor, ME). Schematic diagrams of the protocol followed in these experiments are shown in Figures 4A, 4B, 4C, 4D, 4E and 4F. These figures are referred to in the context of the protocol described below.

For induction of EAE, 300 µg of bMBP and 400 ng of Pertussis toxin (List Biological Technologies, Campbell, CA) were emulsified in complete Freund's adjuvant containing 8 mg/ml of H37Re and injected on either side of the base of the tail (Fig. 4A). Another injection containing 400 ng of Pertussis toxin was administered 48 hours later. The injections induced EAE, which peaked (Fig. 4B) and gradually tapered off, such that eventually, all clinical symptoms of EAE were resolved (Fig. 4C).

SEB was administered one month after resolution of disease (Fig. 4D). The mice were injected i.p. with 3 doses of 10⁵ U of IFNτ 48 hours before, on the day of, and 48 hours after injection of 40 µg SEB (Toxin Technology, Sarasota, FL) and 400 ng of Pertussis toxin (List Biological Technologies, Campbell, CA) (in 0.2 ml PBS) for superantigen reactivation. Control mice received SEB and Pertussis toxin only. The IFNτ preparation was identical to that described in Example 2. Mice were examined daily for signs of EAE and severity of disease was graded as described in Example 2.

**TABLE 5**

| IFN_{T} PREVENTS SUPERANTIGEN REACTIVATION OF EAE | | | | |
|---|---|---|---|---|
| **Treatment** | **No. of IFN Doses** | **Disease Incidence** | **Mean Day of Onset** | **Mean Severity** |
| Expt. 1 | | | | |
| none | 0 | 3/4 | 6.0 ± 1.7 | 1.6 ± 0.5 |
| OvIFNτ | 3 | 0/4 | -- | -- |

| Expt. 2 | | | | |
|---|---|---|---|---|
| none | 0 | 3/5 | 10.0 ± 2.5 | 1.4 ± 0.4 |
| OvIFNτ | 3 | 0/5 | -- | -- |

The data are summarized in Table 5, above. Of a total of nine control mice (receiving no IFNτ), six developed a reactivation of EAE. The mean day of onset was 6 ± 1.7 in the first experiment, and 10 ± 2.5 in the second experiment. Mean severity of the disease was 1.6 ± 0.5 in the first experiment, and 1.4 ± 0.4 in the second experiment. Of the nine animals that were treated with IFNτ, however, none developed symptoms of the disease.

### EXAMPLE 5

### IFNτ Inhibits Vβ-specific T-Cell Activation

The effect of IFNτ treatment of SEB-induced Vβ specific T cell expansion *in vitro* was evaluated as follows. FACS reagents were obtained from Pharmingen, San Diego, CA. Vβ specific T-cell FACS analysis was performed on naive, SEB-injected (50 µg) or IFNτ (10⁵ U) and SEB (50 µg) injected NZW mice. All injections were i.p. and were administered as described in Example 3. Analyses were performed 72 hours after the injections.

For FACS analysis, ~10⁶ T cells were isolated from the animals and incubated with biotin-labeled anti-Vβ antibodies for 45 minutes. The cells were then washed and incubated with strepavidin-phycoerythrin for 15 minutes, followed by another wash and a 45 minute incubation with FITC-labeled anti-CD4 antibodies. The cells were washed again and analyzed on a FACSort (Becton-Dickinson, Mountain View, CA) in duplicate as 10,000 events per sample.

Results of exemplary experiments are shown in Figure 5. Open bars represent naive animals; closed bars represent SEB-injected animals, and crosshatched bars represent IFNτ- and SEB-injected animals. Values are presented as percentage of positively stained cells ± standard error. Values for the Vβ8⁺CD4⁺ T cell subset of SEB-injected and IFNτ- and SEB-injected were significantly different as shown by student's t test (P < 0.02). Naive NZW mice exhibited 5.1 ± 0.1 % Vβ8⁺CD4⁺ T cells. After injection with 50 µg of SEB, this subset was expanded to 10.2 ± 0.2%. When 10⁵ U of IFNτ preceded SEB injection, expansion of the Vβ8⁺CD4⁺ T-subset was limited to 7.6 ± 0.2%. Partial inhibition of Vβ7⁺ and Vβ11⁺ T cells, for which SEB is also specific. was also observed.

These data indicate that treatment with IFNτ can partially inhibit SEB-induced Vβ T cell expansion *in vivo,* and further support the observation that IFNτ inhibits or eliminates symptoms of MBP-induced EAE.

### EXAMPLE 6

### Orally-Administered OvIFNτ Blocks Development of Experimental Allergic Encephalomyelitis

Orally-administered and injected IFN-τ was tested for its ability to prevent the induction of EAE. Recipient New Zealand White (NZW) mice received OvIFNτ (10⁵ U/ml) by either i.p. injection or oral feeding 48 hours prior to, on the day of, and 48 hours after immunization with bMBP for induction of EAE. 10⁵ U of IFNτ were mixed with PBS to a total volume of 100 µl and administered using a feeding tube placed down the esophagus and into the stomach. The dilution of the IFNτ in PBS was done immediately before administration.

For induction of EAE in NZW mice, 300 µg of bovine myelin basic protein (bMBP) was emulsified in complete Freund's adjuvant (CFA) containing 8 mg/ml of H37Ra (Mycobacterium tuberculosis, Difco, Detroit, MI) and injected on either side of the base of the tail. On the day of immunization and 48 hours later, 400 ng of Pertussis toxin (List Biologicals, Campbell, CA) was also injected. For induction of EAE in SJL/J mice, the same protocol was used as described except mice were immunized again 7 days after the initial immunization. Mice were examined daily for signs of EAE and severity of disease was graded as described in Example 2.

Anti-OvIFNτ monoclonal antibody (mAb), HL127, was used to determine whether prevention of EAE was specific to OvIFNτ treatment (antibody HL127, directed against aa 139-172 of SEQ ID NO:2. neutralizes the antiviral activity of OvIFNτ in an antiviral assay using the MDBK cell line). A 1:10 dilution of HL127 was incubated for 2 hours with OvIFNτ prior to administration by either i.p. injection or oral feeding. Antibodies directed against IFNτ antigens, may be generated using the information herein combined with known techniques for antibody production *(e.g.,* Harlow, *et al*., 1988).

The results are shown in Table 6, below. Both oral feeding and i.p. injection of OvIFNτ protected against acute induction of EAE. None of the animals that received IFNτ via i.p. injection developed symptoms of EAE, while of the animals that received IFNτ orally, 7 of 9 (78%) were protected. Antibody HL127 was effective at partially neutralizing the ability of the OvIFNτ to block EAE. These data indicate that orally-administered IFNτ is effective as a treatment in an animal model of multiple sclerosis.

**Table 6**

| Oral Feeding of OvIFNτ Blocks Acute EAE and Can Be Reversed by an OvIFNτ Specific Monoclonal Antibody in NZW Mice | | | | |
|---|---|---|---|---|
| **Route of Admini- stration** | **Treatment** | **Disease Incidence** | **Mean Day of Onset** | **Mean Severity** |
| i.p. | PBS | 4/4 | 24.8 ± 2.1 | 2.5 ± 0 |
| i.p. | OvIFNτ | 0/4 | -- | -- |
| i.p. | OvIFNτ + HL127 | 3/4 | 20.7 ± 1.2 | 2.3 ± 0.6 |
| oral | PBS | 7/9 | 22.0 ± 1.0 | 2.7 ± 0.6 |
| oral | OvIFNτ | 2/9 | 19 | 3 |
| oral | OvIFNτ + HL127 | 5/8 | 20.7 ± 0.6 | 3 ± 0 |
| OvIFNτ (10⁵ U) was administered 48 hours prior to MBP immunization, on the day of MBP immunization and 48 hours after MBP immunization by either i.p. injection or oral feeding. HL127, a monoclonal antibody specific for OvIFNτ, was incubated with OvIFNτ for two hours prior to administration. | | | | |

### EXAMPLE 7

### Detection of OvIFNτ in Sera Following Oral Administration

The amount of OvIFNτ detectable in the sera of mice (treated as above) was compared over time after oral feeding (as above) or i.p. injection of OvIFNτ. Mice were administered 3 × 10⁵ U of OvIFNτ and bled at 0.5, 2, 4, 6, 24 and 48 hours following IFNτ administration. Sera were tested in a cytopathic effect (viral plaque) assay (Familetti, *et al*., 1981) to determine the amount of IFNτ in the samples.

Briefly, dilutions of IFNτ were added to MDBK cells grown to confluency in a flat bottom 96 well plate and incubated for 18 to 24 hours at 37°C. Vesicular stomatatosis virus (VSV) was added to the plate for 45 minutes at room temperature. Virus was removed and methyl cellulose was added and the plate incubated for 48 hours at 37°C. After removal of methyl cellulose, the plate was stained with crystal violet for visualization of plaques. For measurement of IFN neutralization, OvIFNτ at a concentration of 500 U/ml was incubated for 1 hour at 37°C with either sera or HL127. One antiviral unit caused a 50% reduction in destruction of the monolayer, relative to untreated MDBK cells infected with VSV (control plates). All samples were assayed simultaneously to eliminate interassay variability.

As shown in Fig. 6, OvIFNτ was detected at 0.5 hour and 2 hours after oral feeding (filled bars) at levels of 200 U/ml. By comparison, somewhat higher levels of OvIFNτ were detected for over a 24 hour period of time after i.p. injection (open bars). These data show that the above dose of IFNτ can be detected in serum for about two hours following oral administration.

### EXAMPLE 8

### Prevention of Chronic Relapse of Experimental Allergic Encephalomyelitis by Orally-Administered OvIFNτ

The ability of OvIFNτ to prevent paralysis was examined using a chronic-relapsing model of EAE, in which SJL mice immunized with MBP develop a chronic form of the disease where the appearance of symptoms occurs in a relapsing-remitting manner (Zamvil and Steinman, 1990).

EAE was induced in SJL mice essentially as described above. The mice were treated with 10⁵ U of OvIFNτ by either i.p. injection or oral feeding on the day of immunization (day 0) and every 48 hours thereafter for the duration of the experiment. As presented in Figure 7A, SJL mice which were immunized with MBP but did not receive OvIFNτ treatment developed chronic relapsing paralysis with a 5/5 incidence of disease, with a peak mean severity of ~2.5 occurring 14 days after the start of the experiment. In contrast, treatment with OvIFNτ by either i.p. injection or oral feeding (Figures 7B and 7C, respectively) resulted in protection from EAE. Incidence of disease in both OvIFNτ treatment groups was reduced to 1/5 animals, with a mean severity of ~1.0. These data indicate that oral administration of IFNτ can block the development of chronic relapsing EAE, and suggest that orally-administered IFNτ may be as effective as i.p. injection when the IFNτ is fed about every 48 hours over an extended period of time.

### EXAMPLE 9

### Histological Analysis

Histological analyses were performed to determine the extent of lymphocyte infiltration into the CNS of MBP-immunized mice treated with OvIFNτ by oral and i.p. routes. Mice were perfused with 4% paraformaldehyde, vertebral columns were removed and treated with formalin for 2 to 3 days. Spinal cords were dissected out and soaked in 0.5 % sucrose overnight at 4°C. Spinal cord sections were embedded and sections cut in a microtome. Sections were fixed to slides in 4 % paraformaldehyde and stained with cresyl violet for visualization of inflammatory infiltrates.

The results are shown in Figures 8A, 8B and 8C at a final magnification of 222×. Lymphocytic lesions were present in control spinal cord white matter (Fig. 8A). In contrast, no lymphocytic infiltrates were detected in mice treated with OvIFNτ by i.p. injection (Fig. 8B) or oral feeding (Fig. 8C). These data suggest that the protective effect of IFNτ is associated with inhibition of lymphocyte infiltration of the CNS.

### EXAMPLE 10

### Induction of IL10 by Treatment with OvIFNτ

During the course of OvIFNτ treatment of SJL for prevention of chronic relapsing EAE, mice were bled and sera were examined for the presence of interleukin 10 (IL10). Sera from mice which received either a single IFNτ (10⁵ U) treatment (by i.p. injection or oral feeding), prolonged IFNτ (10⁵ U) treatment (by i.p. injection or oral treatment for greater than 20 days) or no treatment were examined for IL10 by enzyme-linked immunosorbent assay (ELISA) using IL10 ELISA kits (Genzyme, Cambridge, MA) following the manufacturer's instructions. All sera samples were tested in duplicate.

No IL10 was detected in control mice or in mice which received a single treatment of OvIFNτ by either i.p. injection or oral feeding. In contrast, SJL mice which received OvIFNτ by either i.p. injection or oral feeding every 48 hours for greater than 20 days had detectable levels of IL 10 in their sera (Figure 9). These data suggest that IFNτ-induced production of IL10 may be a contributing mechanism by which OvIFNτ prevents development of EAE.

### EXAMPLE 11

### Cessation of Treatment with OvIFNτ Results in Relapsing Paralysis

SJL mice which were protected from EAE by OvIFNτ treatment via i.p. injection or oral feeding (every 48 hours) were followed for 58 days, during which time no disease development was observed. Treatment with OvIFNτ was then removed and the mice were observed for an additional 22 days for symptoms of disease.

The results are shown in Figure 10. IFNτ treatment is denoted as plus signs and removal of IFNτ treatment is denoted as minus signs beneath the graph. Disease incidence in each treatment group was as follows: PBS control=3/4 (square); i.p. injection=3/3 (triangle); oral feeding =:3/4 (circle).

Both groups of mice which had previously been protected from EAE by OvIFNτ treatment developed signs of paralysis 6 to 12 days after removal of the OvIFNτ treatment. These data indicate that ongoing administration of IFNτ, by either i.p. injection or oral feeding, is desirable for continued protection from EAE in the chronic-relapsing model of EAE.

### EXAMPLE 12

### Oral Administration of OvIFNτ Reduces Anti-OvIFNτ Antibody Response

After removal of OvIFNτ treatment in the experiments described in Example 11, above, mice from each treatment group were bled and sera were examined for the presence of anti-OvIFNτ antibodies (Ab). The antigen, OvIFNτ, was adsorbed to the flat bottoms of plastic tissue culture wells overnight at a concentration of 600 ng/well, and subsequently evaporated to dryness. The plates were treated with 5 % milk (Carnation) in PBS for 2 hours in order to block nonspecific binding and then washed 3 times with PBS containing 0.05% Tween 20. Various dilutions of sera from mice which were untreated, OvIFNτ treated by i.p. injection and OvIFNτ treated by oral feeding were added and incubated for 3 hours. Binding was assessed with goat anti-mouse immunoglobulin coupled to horseradish peroxidase. Color development was monitored at 492 nm in an ELISA plate reader (Bio-Rad, Richmond. CA) after o-phenylenediamine and H₂O₂ were added and the reaction terminated with 2M H₂SO₄.

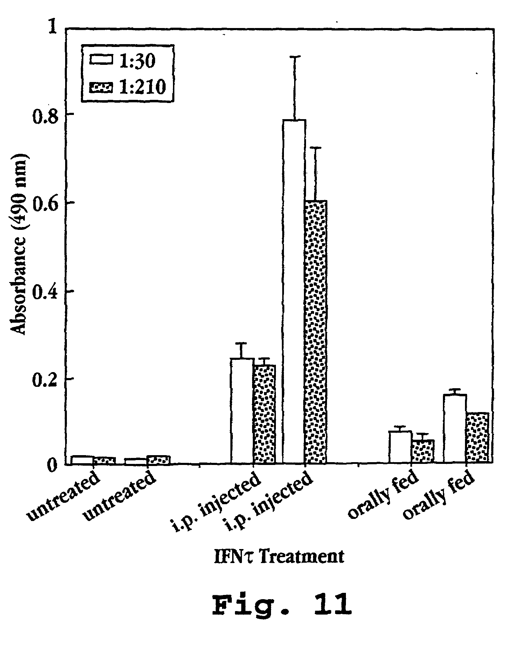

Exemplary results are shown in Figure 11. Sera from untreated, OvIFNτ treated-i.p. injected and OvIFNτ treated-orally fed (2 mice/group) were examined by ELISA using multiple dilutions, including 1:30 (open bars) and 1:120 (filled bars). Mice which received OvIFNτ by oral feeding exhibited minimal Ab levels while mice which received OvIFNτ by i.p. injection exhibited elevated levels of anti-OvIFNτ Ab. As expected, mice which received no OvIFNτ treatment displayed no anti-OvIFNτ Ab.

Sera were also examined for their ability to neutralize OvIFNτ antiviral activity on MDBK cells as described above. The results are shown in Table 7, below. None of the sera from either i.p. injected or orally fed mice possessed neutralizing activity. These data suggest that oral treatment with IFNτ circumvents the Ab response directed against OvIFNτ protein observed in i.p. injection-treated individuals, and that neither treatment typically results in the generation of neutralizing antibodies.

**Table 7**

| Sera from Mice Treated with OvIFNτ by i.p. Injection or Oral Feeding do Not Possess Neutralizing Activity | |
|---|---|
| **500 U/ml of OvIFN**τ **Cocultured with Sera From:** | **OvIFN**τ **Titer (U/ml)** |
| untreated | 500 |
| i.p. injected | 500 |
| orally fed | 500 |
| HL127 | <50 |

While the invention has been described with reference to specific methods and embodiments, it is appreciated that various modifications and changes may be made without departing from the invention.

## Claims

1. Use of tau-interferon (TENτ) for the manufacture of a medicament for treating a disease responsive to treatment by IFNτ by orally administering said medicament to a mammalian subject.

2. The use acccording to claim 1, wherein said disease is an autoimmune disease.

3. The use according to Claim 1 or 2, wherein said
IFNτ is selected from the group consisting of ovine IFNτ(OvIFNτ) and bovine IFNτ.

4. The use according to claim 3, wherein said OvIFNτ has the sequence represented as SEQ ID NO:2.

5. The use according to any one of claims 1 to 4, wherein said IFNτ is a recombinantly produced IFNτ.

6. The use according to any one of claims 2 to 5, wherein said autoimmune disease is selected from Type I diabetes mellitus, rheumatoid arthritis, lupus erythematosus, psoriasis and multiple sclerosis.

7. The use according to any one of claims 1 to 6, wherein said mammalian subject is a human subject.

8. The use according to any one of claims 1 to 7, wherein said Interferon-τ is administered in an amount between about 1 x 10⁵ and about 1 x 10⁸ units of IFNτ per day.

9. The use according to claim 8, wherein said Interferon-τ is administered in an amount between about 1 x 10⁶ and about 1 x 10⁷ units of IFNτ per day.

10. The use according to any one of claims 1 to 9, wherein said administering further includes administering a second autoimmune disease treatment agent.

11. The use according to claim 10, wherein said second agent is a corticosteroid drug.

## Patentansprüche

1. Verwendung von tau-Interferon (IFNτ) zur Herstellung eines Medikaments zur Behandlung einer Erkrankung, die auf eine Behandlung mit IFNτ anspricht, bei der das Medikament einem Säugerindividuum oral verabreicht wird.

2. Verwendung nach Anspruch 1, wobei die Erkrankung eine Autoimmunerkrankung ist.

3. Verwendung nach Anspruch 1 oder 2, wobei das IFNτ ausgewählt ist aus der Gruppe bestehend aus ovinem IFNτ (OvIFNτ) und bovinem IFNτ.

4. Verwendung nach Anspruch 3, wobei das OvIFNτ die als SEQ ID Nr. 2 dargestellte Sequenz besitzt.

5. Verwendung nach einem der Ansprüche 1 bis 4, wobei das IFNτ ein rekombinant hergestelltes IFNτ ist.

6. Verwendung nach einem der Ansprüche 2 bis 5, wobei die Autoimmunerkrankung ausgewählt ist aus Typ-I-Diabetes mellitus, rheumatoide Arthritis, Lupus erythematodes, Schuppenflechte und multipler Sklerose.

7. Verwendung nach einem der Ansprüche 1 bis 6, wobei das Säugerindividuum ein menschliches Individuum ist.

8. Verwendung nach einem der Ansprüche 1 bis 7, wobei das Interferon-τ in einer Menge zwischen etwa 1 x 10⁵ und etwa 1 x 10⁸ Einheiten IFNτ pro Tag verabreicht wird.

9. Verwendung nach Anspruch 8, wobei das Interferon-τ in einer Menge zwischen etwa 1 x 10⁶ und etwa 1 x 10⁷ Einheiten IFNτ pro Tag verabreicht wird.

10. Verwendung nach einem der Ansprüche 1 bis 9, wobei die Verabreichung weiterhin die Verabreichung eines zweiten Mittels zur Behandlung einer Autoimmunerkrankung einschließt.

11. Verwendung nach Anspruch 10, wobei das zweite Mittel ein Corticosteroid ist.

## Revendications

1. Utilisation d'interféron tau (IFNτ) pour préparer un médicament destiné au traitement d'une maladie sensible à un traitement par l'IFNτ par administration orale dudit médicament à un patient mammifère.

2. Utilisation selon la revendication 1, pour laquelle ladite maladie est une maladie auto-immune.

3. Utilisation selon la revendication 1 ou 2, pour laquelle ledit IFNτ est choisi dans l'ensemble comprenant l'IFNτ ovin (OVIFNτ) et l'IFNτ bovin.

4. Utilisation selon la revendication 3, pour laquelle ledit OvIFNτ a la séquence SEQ ID NO:2.

5. Utilisation selon l'une quelconque des revendications 1 à 4, pour laquelle ledit IFNτ est un IFNτ obtenu par recombinaison.

6. Utilisation selon l'une quelconque des revendications 2 à 5, pour laquelle ladite maladie auto-immune est choisie parmi le diabète sucré de type I, la polyarthrite rhumatoïde, le lupus érythémateux, le psoriasis et la sclérose en plaque.

7. Utilisation selon l'une quelconque des revendications 1 à 6, pour laquelle ledit patient mammifère est un patient humain.

8. Utilisation selon l'une quelconque des revendications 1 à 7, pour laquelle ledit interféron τ est administré en une quantité comprise entre environ 1 x 10⁵ et environ 1 x 10⁸ unités d'IFNτ par jour.

9. Utilisation selon la revendication 8, pour laquelle ledit interféron τ est administré en une quantité comprise entre environ 1 x 10⁶ et environ 1 x 10⁷ unités d'IFNτ par jour.

10. Utilisation selon l'une quelconque des revendications 1 à 9, pour laquelle ladite administration comprend en outre l'administration d'un deuxième agent de traitement des maladies auto-immunes.

11. Utilisation selon la revendication 10, pour laquelle ledit agent est un médicament corticostéroïdien.
